(19) Europäisches Patentamt
European Patent Office
Office européen des brevets

(11) Numéro de publication: **0 040 573**
**B1**

(12) # FASCICULE DE BREVET EUROPEEN

(45) Date de publication du fascicule du brevet: **01.08.84**

(21) Numéro de dépôt: **81400783.7**

(22) Date de dépôt: **19.05.81**

(51) Int. Cl.³: **C 07 D 417/12**, C 07 D 401/12, C 07 D 409/12, C 07 D 413/12, C 07 D 215/56, A 61 K 31/47 // C07D265/22, C07D277/46, C07D263/48, C07D233/88

(54) **Dérivés de l'acide 4-hydroxy 3-quinoléine carboxylique substitués en 2, leur préparation, leur application comme médicament, les compositions les renfermant et les intermédiaires nouveaux obtenus.**

(30) Priorité: **19.05.80 FR 8011100**

(43) Date de publication de la demande: **25.11.81 Bulletin 81/47**

(45) Mention de la délivrance du brevet: **01.08.84 Bulletin 84/31**

(84) Etats contractants désignés: **AT BE CH DE FR GB IT LI LU NL SE**

(56) Documents cités:
**EP - A - 0 012 639**
**FR - A - 2 340 735**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Allais, André, 1, allée Eugénie, F-93220 Gagny (FR)**
Inventeur: **Clemence, François, 2, rue Turgot, F-75009 Paris (FR)**
Inventeur: **Deraedt, Roger, 23, allée Jean-Baptiste Clément, Les Pavillons-sous-Bois (FR)**
Inventeur: **Le Martret, Odile, 42, avenue de Versailles, F-75016 Paris (FR)**

(74) Mandataire: **Tonnellier, Marie-José et al, 102, route de Noisy Boîte postale no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne des dérivés de l'acide 4-hydroxy 3-quinoléïne carboxylique substitués en 2, leur procédé de préparation, leur application comme médicament, les compositions les renfermant et les nouveaux intermédiaires obtenus.

L'invention a pour objet les composés de formule (I')

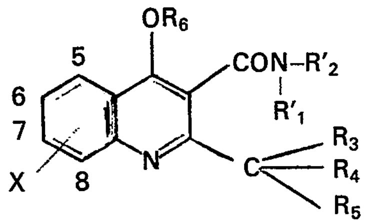

(I)

dans laquelle X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone un radical trifluorométhyle, un radical trifluorométhylthio ou un radical trifluorométhoxy, $R_1'$ représente un atome d'hydrogène ou un radical alcoyle renfermant de 1 à 4 atomes de carbone, $R_2'$ représente un atome d'hydrogène ou un cycle saturé ou insaturé, pouvant contenir un ou plusieurs hétéroatomes choisis parmi le soufre, l'azote et l'oxygène et, le cas, échéant, substitué par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyle et g) le radical nitro, ou $R_1'$ et $R_2'$ forment ensemble l'un quelconque des cycles saturés ou insaturés mentionnés ci-dessus, ledit cycle étant alors relié à l'atome d'azote par une double liaison, $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que $R_3$, $R_4$ et $R_5$ ne représentent pas en même temps chacun un atome de fluor et $R_6$ représente un atome d'hydrogène, un radical alcoyle renfermant de 1 à 8 atomes de carbone ou un radical acyle renfermant de 2 à 8 atomes de carbone, ainsi que leurs sels d'addition avec les acides et les bases.

Lorsque $R_1'$ représente un radical alcoyle, il s'agit de préférence du radical méthyle ou éthyle.

Lorsque X représente un atome d'halogène, il s'agit de préférence d'un atome de chlore.

Lorsque X représente un radical alcoyle, il s'agit de préférence du radical méthyle, éthyle, n-propyle, n-butyle, n-pentyle, isopropyle ou isobutyle.

Lorsque X représente un radical alcoxy, il s'agit de préférence du radical méthoxy, éthoxy ou n-propoxy.

Lorsque $R_2'$ représente un cycle, il s'agit de préférence d'un cycle thiazolyle, phényle, pyridinyle, thiényle, benzothiazolyle, oxazolyle ou imidazolyle.

Lorsque $R_2'$ représente un cycle substitué, le ou les substituants sont de préférence choisis dans le groupe constitué par l'atome de chlore, le radical méthyle, le radical éthyle, le radical diméthylaminométhyle, le radical phényle, le radical méthoxy, le radical éthoxy, le radical hydroxy, le radical trifluorométhyle et le radical nitro.

Lorsque $R_3$ représente un radical alcoyle, il s'agit de préférence du radical méthyle ou éthyle.

Lorsque $R_3$ représente un atome d'halogène, il s'agit de préférence d'un atome de fluor, de chlore ou de brome.

$R_4$ représente de préférence un atome de fluor, de chlore ou de brome.

$R_5$ représente de préférence un atome de chlore ou de brome.

Lorsque $R_6$ représente un radical alcoyle, il s'agit de préférence du radical méthyle ou éthyle.

Lorsque $R_6$ représente un radical acyle, il s'agit de préférence du radical acétyle, propionyle ou butynyle.

Parmi les sels d'addition avec les acides, on peut citer ceux formés avec les acides minéraux tels que les acides chlorhydrique, bromhydrique, sulfurique ou phosphorique ainsi que ceux formés avec les acides sulfoniques tels que les acides alcoyl ou arylsulfoniques, par exemple l'acide méthanesulfonique ou paratoluènesulfonique.

Parmi les sels d'addition avec les bases, on peut citer ceux formés avec les métaux alcalins comme le sodium et le potassium et les amines, par exemple, la triméthylamine ou la diméthylamine.

L'invention a notamment pour objet les composés de formule (I') telle que définie précédemment, répondant à la formule (I)

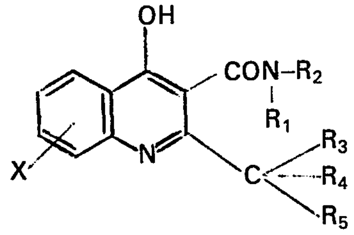

(I)

dans laquelle X est défini comme précédemment, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle ou tétrazolyle, éventuellement substitué par un radical alcoyle renfermant de 1 à 4 atomes de carbone, et phényle, éventuellement substitué par au moins un radical choisi dans le groupe formé par les radicaux hydroxy, les radicaux alcoyles renfermant de 1 à 4 atomes de carbone, les radicaux alcoxy renfermant de 1 à 4 atomes de

carbone, le radical trifluorométhyle, le radical nitro et les atomes d'halogène et $R_3$, $R_4$ et $R_5$ sont définis comme précédemment, ainsi que leurs sels d'addition avec les acides et les bases.

L'invention a plus particulièrement pour objet, les composés de formule I tels que définis précédemment, pour lesquels $R_3$ représente un atome d'hydrogène, un radical alcoyle ou le même atome d'halogène que $R_5$, $R_4$ représente un atome d'hydrogène ou le même atome d'halogène que $R_5$, ainsi que leurs sels d'addition avec les acides et les bases.

L'invention a notamment pour objet les composés de formule (I) pour lesquels X représente un radical trifluorométhyle, ainsi que leurs sels d'addition avec les acides et les bases, et ceux pour lesquels $R_1$ représente un atome d'hydrogène ainsi que leurs sels d'addition avec les acides et les bases.

L'invention a notamment pour objet les composés de formule (I) pour lesquels $R_3$ et $R_4$, identiques ou différents, représentent un atome d'hydrogène ou un atome de chlore et $R_5$ représente un atome de chlore, ainsi que leurs sels d'addition avec les acides et les bases, et plus particulièrement parmi ceux-ci, les composés pour lesquels le radical 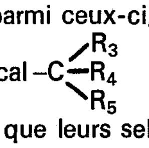 représente le radical $-CHCl_2$, ainsi que leurs sels d'addition avec les acides et les bases.

Parmi les composés de l'invention, on peut citer tout particulièrement les composés pour lesquels $R_2$ représente le radical thiazolyle, ainsi que leurs sels d'addition avec les acides et les bases.

Comme composé préféré de l'invention, on peut citer, le 2-(dichlorométhyl) 4-hydroxy N-(2-thiazolyl) 8-trifluorométhyl 3-quinoléïne carboxamide, ainsi que ses sels d'addition avec les acides et les bases.

Les composés de formule (I') ainsi que leurs sels, présentent d'intéressantes propriétés pharmacologiques. Ils présentent notamment une activité analgésique remarquable et une très faible activité antiinflammatoire, source d'une bonne tolérance au niveau gastro intestinal.

Ces propriétés justifient l'utilisation des produits de formule (I') ainsi que de leurs sels d'addition avec les acides et les bases thérapeutiquement compatibles.

L'invention a donc pour objet les composés de formule (I') tels que définis ci-dessus, ainsi que leurs sels avec les acides et les bases thérapeutiquement compatibles, à titre de médicaments.

L'invention a plus particulièrement pour objet, à titre de médicaments, le composé de l'exemple 1, ainsi que ses sels d'addition avec les acides et les bases thérapeutiquement acceptables.

Les médicaments, objet de l'invention, peuvent être utilisés dans le traitement des algies musculaires, articulaires ou nerveuses, des douleurs dentaires, des migraines ainsi que dans le traitement des affections rhumatismales.

L'invention s'étend aux compositions pharmaceutiques renfermant comme principe actif, les medicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

L'invention a également pour objet un procédé de préparation des composés de formule (I'), caractérisé en ce que l'on soumet un composé de formule (II)

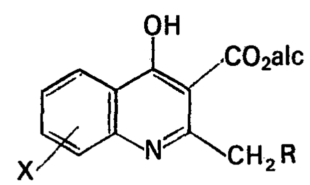

(II)

dans laquelle X conserve la même signification que précédemment, alc représente un radical alcoyle renfermant de 1 à 8 atomes de carbone, et R représente un atome d'hydrogène ou un radical alcoyle renfermant de 1 à 4 atomes de carbone, à l'action d'un agent d'halogénation, puis, le cas échéant, à l'action d'un second agent d'halogénation, différent du premier, pour obtenir le composé de formule ($III_A$)

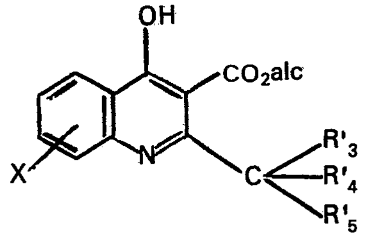

($III_A$)

dans laquelle $R_3'$, $R_4'$ et $R_5'$ conservent la signification donnée pour $R_3$, $R_4$ et $R_5$, que l'on soumet, si désiré, à l'action d'un composé de formule $Hal_1M$, dans laquelle $Hal_1$ représente un atome d'halogène et M un atome de métal alcalin, pour obtenir le composé de formule ($III_B$)

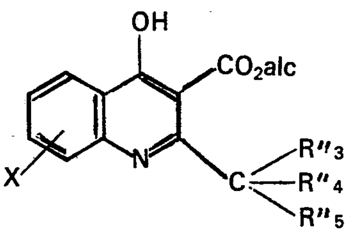

($III_B$)

dans laquelle $R_3''$ représente un atome d'hydrogène, un atome d'halogène $Hal_1$, un radical alcoyle renfermant de 1 à 4 atomes de carbone, $R_4''$ représente un atome d'hydrogène ou un atome d'halogène $Hal_1$ et $R_5''$ représente un atome d'halogène $Hal_1$, puis soumet le composé de formule ($III_A$) ou le composé de formule ($III_B$) à l'action d'un agent de saponification pour obtenir un acide correspondant de formule (IV)

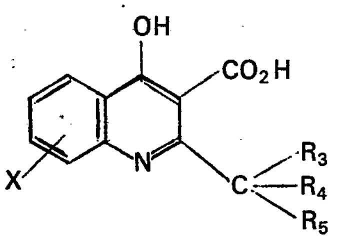

(IV)

dans laquelle $R_3$, $R_4$ et $R_5$ conservent leur signification précédente, que l'on transforme, si désiré, en dérivé fonctionnel d'acide, puis soumet l'acide de formule (IV) ou un dérivé fonctionnel de cet acide, à l'action d'un composé de formule (V)

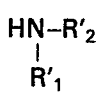

(V)

dans laquelle $R_1'$ et $R_2'$ conservent leur signification précédente, pour obtenir le composé de formule ($I_A'$) correspondant

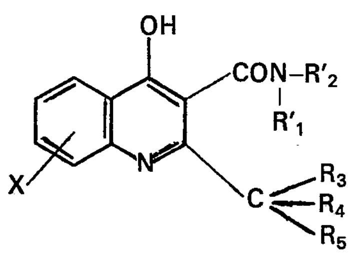

($I'_A$)

dans laquelle X, $R_1'$, $R_2'$, $R_3$, $R_4$ et $R_5$ sont définis comme précédemment, correspondant à un produit de formule (I') dans laquelle $R_6$ représente un atome d'hydrogène, produit de formule ($I_A'$) que l'on soumet, si désiré, à l'action d'un agent d'éthérification ou d'estérification, pour obtenir un produit de formule ($I_B'$)

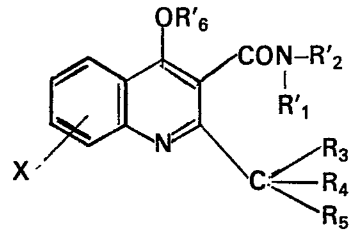

($I'_B$)

dans laquelle X, $R_1'$, $R_2'$, $R_3$, $R_4$ et $R_5$ sont définis comme précédemment et $R_6'$ a les valeurs indiquées précédemment pour $R_6$, à l'exception d'hydrogène, produit de formule ($I_A'$) ou ($I_B'$) que, si désiré, l'on soumet à l'action d'un acide ou d'une base pour en former le sel.

L'invention a plus particulièrement pour objet un procédé selon ce qui précède, caractérisé en ce que l'on soumet un composé de formule ($II_a$)

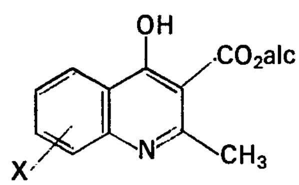

($II_a$)

dans laquelle X et alc conservent leur signification précédente, à l'action d'un agent d'halogénation pour obtenir le composé de formule ($III_a$)

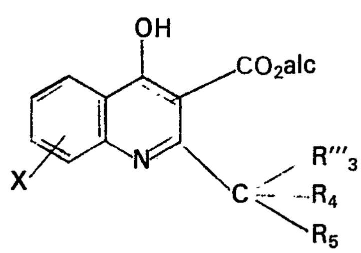

($III_a$)

dans laquelle $R_3'''$ et $R_4$ représentent, soit un atome d'hydrogène, soit le même atome d'halogène que $R_5$, produit ($III_a$), que l'on soumet à l'action d'un agent de saponification, pour obtenir un composé de formule ($IV_a$)

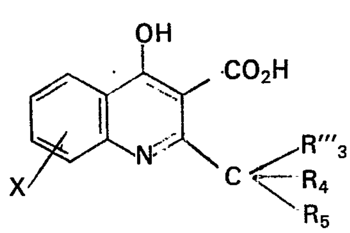

($IV_a$)

dans laquelle X, $R_3'''$, $R_4$ et $R_5$ sont définis comme précédemment, que l'on transforme, si désiré, en dérivé fonctionnel d'acide, puis soumet soit l'acide de formule ($IV_a$), soit un dérivé fonctionnel de cet acide, à l'action d'un composé de formule (V)

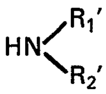

(V)

dans laquelle $R_1'$ et $R_2'$ conservent leur signification précédente, pour obtenir un composé de formule ($I_C'$)

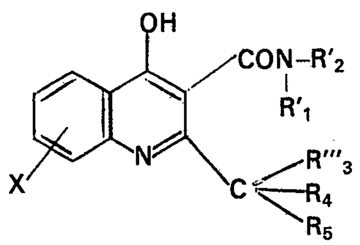

($I'_c$)

dans laquelle X, $R_1'$, $R_2'$, $R_3'''$, $R_4$ et $R_5$ sont définis comme précédemment, correspondant à un produit de formule (I') dans laquelle $R_6$ représente un atome d'hydrogène et $R_3$ a les valeurs de $R_3'''$, produit de formule ($I_C'$) que l'on soumet, si désiré, à l'action d'un agent d'éthérification ou d'estérification, pour obtenir un produit de formule ($I_D'$)

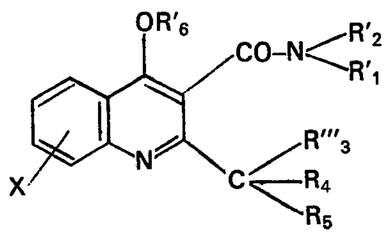

($I'_D$)

dans laquelle X, $R_1'$, $R_2'$, $R_3'''$, $R_4$ et $R_5$ sont définis comme précédemment et $R_6'$ a les valeurs indiquées précédemment pour $R_6$, à l'exception d'hydrogène, produit de formule ($I_C'$ ou $I_D'$) que l'on soumet, si désiré, à l'action d'un acide ou d'une base pour en former le sel.

Comme agent d'halogénation, on peut utiliser les halogènes, les composés du type $Cu(Hal)_2$ dans lesquels Hal représente un atome d'halogène, ou les N-bromo ou N-chloroamides comme par exemple le N-bromo ou N-chloro succinimides ou acétamides.

Il va de soi que selon que l'on cherche à obtenir des dérivés mono, di ou trihalogénés, on mettra on œuvre des quantités d'agents d'halogénation différentes.

Le dérivé fonctionnel d'acide utilisé est un chlorure d'acide, un ester d'alcoyle inférieur, un anhydride ou un anhydride mixte.

La condensation de l'acide ou du dérivé fonctionnel d'acide avec le composé de formule (V) a lieu au sein d'un solvant inerte, comme par exemple, le benzène, le toluène, la pyridine ou l'acétate d'éthyle en présence d'un agent basique et de préférence en présence de triéthylamine.

Les réactions d'éthérification et d'estérification des produits de formule ($I_A'$ et $I_C'$) sont effectuées selon les méthodes usuelles.

Dans un mode de réalisation préférée du procédé de l'invention,

- alc représente un radical méthyle ou éthyle;
- l'agent d'halogénation est un N-haloamide comme le N-chloro ou N-bromo acétamide ou le N-chloro ou N-bromo succinimide, que l'on utilise au sein d'un solvant comme le tétrachlorure de carbone, le chloroforme ou l'acide acétique, en présence d'initiateurs radicalaires comme les peroxydes, par exemple le peroxyde de benzoyle, comme l'azobisisobutyronitrile ou comme la lumière,
- $Hal_1$ représente un atome de fluor;
- M représente un atome de potassium;
- l'agent de saponification est la soude ou la potasse.

L'invention a plus particulièrement pour objet un procédé tel que défini précédemment, caractérisé en ce que l'agent d'halogénation utilisé est le N-chloro ou le N-bromosuccinimide.

L'invention a également pour objet un procédé de préparation des produits de formule (I') telle que définie précédemment, caractérisé en ce que l'on soumet le composé de formule ($III_A$), ($III_B$) ou ($III_a$) tel que décrit précédemment, à l'action d'un composé de formule (V)

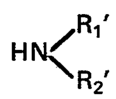

(V)

dans laquelle $R_1'$ et $R_2'$ sont définis comme précédemment, en présence d'un trialcoylaluminium, pour obtenir un composé de formule ($I_A'$) ou ($I_C'$), telles que définies précédemment, puis, si désiré, poursuit la synthèse comme décrit précédemment.

Dans un mode de réalisation préférée du procédé de l'invention, le trialcoylaluminium est un triméthyl ou un triisobutylaluminium.

L'invention a également pour objet un procédé de préparation des composés de formule (I'), telle que définie précédemment, caractérisé en ce que l'on soumet un composé de formule (VI)

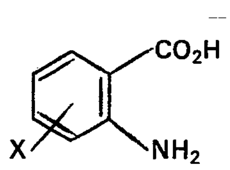

(VI)

dans laquelle X conserve sa signification précédente, à l'action d'un acide de formule (VII)

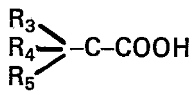
(VII)

dans laquelle $R_3$, $R_4$, $R_5$ conservent leurs significations précédentes ou d'un dérivé fonctionnel de l'acide de formule (VII), pour obtenir un composé de formule (VIII)

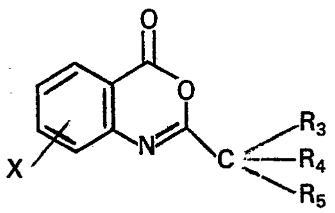
(VIII)

dans laquelle X conserve sa signification précédente, que l'on soumet à l'action d'un composé de formule (IX)

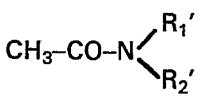
(IX)

dans laquelle $R_1'$ et $R_2'$ conservent leurs significations précédentes, pour obtenir le composé de formule (X)

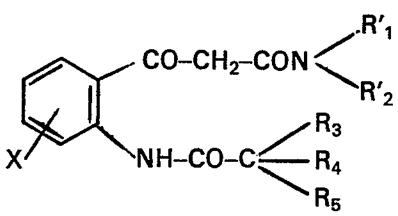
(X)

dans laquelle $R_1'$, $R_2'$, $R_3$, $R_4$ et $R_5$ conservent leurs significations précédentes, que l'on cyclise en présence d'un agent alcalin, pour obtenir un composé de formule $I_A'$, telle que définie précédemment, composé de formule ($I_A'$) que l'on soumet, si désiré, à l'action d'un agent d'éthérification ou d'estérification, pour obtenir un composé de formule ($I_B'$), telle que définie précédemment, composé de formule ($I_A'$) ou ($I_B'$) que, si désiré, l'on soumet à l'action d'un acide ou d'une base pour en former le sel.

Dans un mode de réalisation préférée du procédé de l'invention:

– le dérivé fonctionnel de l'acide de formule (VII) est un halogénure ou un anhydride;

– la réaction entre le composé de formule (VIII) et le composé de formule (IX) a lieu en présence d'un organolithien ou d'un amidure de lithium, par exemple en présence de butyllithium ou de diisopropylamidure de lithium;

– la cyclisation du composé de formule (X) est effectuée en présence d'un agent alcalin, tel qu'un hydrure ou un carbonate alcalin, ou une amine, par exemple en présence d'hydrure de sodium, de carbonate de sodium ou de potassium, de pipéridine, de 4-aminopyridine, le triéthylamine, de 1,5-diazabicyclo [4,30] non-5-ène, de 1,4-diazabicyclo [2,2,2] octane ou de 1,5-diazabicyclo [5,4,0] undéc-5-ène.

Les composés de formule (II) et (VI) utilisés comme produits de départ du procédé de l'invention sont des produits connus d'une façon générale, qui peuvent être préparés selon les procédés indiqués dans la demande de brevet français n° 2 340 735 ou dans le brevet français n° 2 157 874.

Les composés de formule ($III_A$), ($III_B$), (IV) et notamment les composés de formule ($III_a$) et ($IV_a$), sont des produits chimiques nouveaux, l'invention a donc pour objet ces produits à titre de produits industriels nouveaux, notamment à titre de produits intermédiaires.

Les exemples suivants illustrent l'invention sans toutefois la limiter.

Exemple 1

2-dichlorométhyl) 4-hydroxy N-(2-thiazolyl) 8-trifluorométhyl 3-quinoléïne carboxamide.

Stade A: 2-dichlorométhyl 4-hydroxy 8-trifluorométhyl 3-quinoléïne carboxylate d'éthyle.

On porte au reflux pendant 24 heures un mélange de 11,92 g de 2-méthyl 4-hydroxy 8-trifluorométhyl 3-quinoléïne carboxylate d'éthyle, 400 $cm^3$ de tétrachlorure de carbone, 13,30 g de N-chloro succinimide et 600 mg de péroxyde de benzoyle. On refroidit le mélange réactionnel à la température ambiante, filtre l'insoluble et le lave au tétrachlorure de carbone. On concentre le filtrat sous pression réduite. On obtient 17 g d'une huile que l'on purifie par chromatographie sur silice en éluant au chlorure de méthlène. On obtient ainsi 13,6 g de produit fondant vers 80–85 °C que l'on empâte dans l'éther de pétrole (eb = 60 °C–80 °C).

On essore, lave et sèche. On obtient ainsi 8,8 g du produit recherché fondant à 88 °C.

Stade B: acide 2-(dichlorométhyl) 4-hydroxy 8-trifluorométhyl 3-quinoléïne carboxylique.

On agite 36 heures à la température ambiante une solution renfermant 22 g du produit préparé au stade A, 220 $cm^3$ d'éthanol et 110 $cm^3$ de lessive de soude. On chasse l'éthanol sous pression réduite à moins de 40 °C. On dilue la solution avec 200 $cm^3$ d'eau, la glace et l'amène à pH = 1 par addition d'acide chlorhydrique concentré. On essore, lave le précipité obtenu à l'eau et le dissout dans l'éther éthylique. On filtre, sèche et concentre sous pression réduite. On obtient ainsi 18 g du produit recherché que l'on utilise tel quel dans le stade suivant.

Stade C: 2-(dichlorométhyl) 4-hydroxy N-(2-thiazolyl) 8-trifluorométhyl 3-quinoléïne carboxamide.

Stade a: préparation du chlorure d'acide.

On porte au reflux une solution renfermant 15 g

du produit préparé au stade précédent, 400 $cm^3$ de benzène anhydre et 16 $cm^3$ de chlorure de thionyle. On maintient le reflux pendant 1 h 30 minutes. On chasse le benzène et l'excès de chlorure de thionyle par distillation sous pression réduite. On obtient un produit que l'on utilise tel quel dans le stade suivant.

Stade b: 2-dichlorométhyl 4-hydroxy N-(2-thiazolyl) 8-trifluorométhyl 3-quinoléïne carboxamide.

On ajoute sous agitation et atmosphère inerte une solution renfermant 4,41 g de 2-aminothiazole, 50 $cm^3$ d'acétate d'éthyle anhydre et 18,5 $cm^3$ de triéthylamine dans une solution renfermant le produit obtenu au stade précédent dans 120 $cm^3$ d'acétate d'éthyle. On porte le mélange réactionnel au reflux pendant une heure, puis abandonne une nuit à la température ambiante. On élimine le chlorhydrate de triéthylamine formé par filtration, on lave le filtrat à l'eau contenant du Cl Na pour éviter les émulsions.

On sèche, concentre à sec sous pression réduite. On concrétise dans 150 $cm^3$ d'acide chlorhydrique à 20%. On essore, lave à l'eau jusqu'à neutralité, puis dissout le précipité encore humide dans 50 $cm^3$ de soude normale. On filtre, acidifie le filtrat à pH 4–5 avec l'acide chlorhydrique à 50%. On glace, essore, lave à l'eau, sèche sous pression réduite à 90 °C, et obtient 8,5 g du produit attendu sous forme brute que l'on recristallise dans l'acétate d'éthyle. On obtient 5,7 g du produit recherché fondant à 204 °C.

Exemple 2

2-dichlorométhyl 4-hydroxy N-phényl 8-trifluorométhyl 3-quinoléïne carboxamide.

On dissout 3,72 g d'aniline dans 100 $cm^3$ de chlorure de méthylène puis ajoute à +15, +18 °C, 17,5 $cm^3$ d'une solution à 25% de triméthylaluminium dans l'hexane et maintient ensuite sous agitation pendant 15 minutes. On ajoute ensuite à la même température 7,56 g de produit obtenu au stade A de l'exemple 1, puis porte au reflux pendant 22 heures. On verse ensuite le mélange dans 100 $cm^3$ d'une solution aqueuse glacée à 20% d'acide chlorhydrique, décante, extrait la phase aqueuse à l'acétate d'éthyle, lave les phases organiques réunies à l'eau, les sèche et évapore le solvant. Les cristaux obtenus sont empâtés dans l'éther éthylique puis séchés et recristallisés dans l'acétate d'éthyle. On obtient 1,1 g de produit attendu que l'on peut purifier à nouveau dans l'éthanol. F=200–202 °C (Décomposition).

Analyse: $C_{18}H_{11}N_2O_2Cl_2F_3$=415,217

| | | | | |
|---|---|---|---|---|
| Calculé: | C% 52,07 | H% 2,67 | N% 6,75 | Cl% 17,08 |
| | F% 13,73 | | | |
| Trouvé: | 52,0 | 2,6 | 6,7 | 17,4 |
| | 13,6 | | | |

Exemple 3

2-dichlorométhyl 4-hydroxy N-(2-pyridinyl) 8-trifluorométhyl 3-quinoléine carboxamide.

On opère de manière analogue à celle décrite à l'exemple 2, au départ de 3,76 g de 2-aminopyridine et de 7,36 g de produit obtenu au Stade A de l'exemple 1.

On obtient après recristallisation du produit dans l'acétate d'éthyle 2,7 g de produit attendu que l'on peut purifier à nouveau dans le méthanol. F=212 °C. (Décomposition).

Analyse: $C_{17}H_{10}N_3Cl_2F_3O_2$=416,199

| | | | | |
|---|---|---|---|---|
| Calculé: | C% 49,06 | H% 2,42 | N% 10,10 | Cl% 17,04 |
| | F% 13,69 | | | |
| Trouvé: | 49,0 | 2,3 | 10,0 | 17,2 |
| | 13,2 | | | |

Exemple 4

2-dichlorométhyl 4-hydroxy N-(2-thiazolyl) 8-trifluorométhyl 3-quinoléïne carboxamide.

On dissout 13,75 g de 2-aminothiazole dans 150 $cm^3$ de toluène puis ajoute à +5 °C, une solution renfermant 30 $cm^3$ de triméthylaluminium à 25% dans l'hexane et 30 $cm^3$ de toluène. On maintient sous agitation à +5, +10 °C pendant une demi-heure puis ajoute en une fois 9,2 g de produit obtenu au stade A de l'exemple 1. Après la fin du dégagement gazeux, on porte la température à +80, +85 °C rapidement et sous gaz inerte pendant 3 heures et demi. On concentre ensuite à sec puis le résidu est additionné de 500 $cm^3$ d'acide chlorhydrique 1N et agité pendant 1 heure. On filtre, lave à l'eau puis avec un mélange d'eau et d'hydroxyde de sodium 2N et enfin à l'eau. On réunit les phases alcalines, filtre puis ajoute lentement au filtrat de l'acide chlorhydrique 2N jusqu'à pH 5. On essore le précipité obtenu, le lave à l'eau et le sèche. On obtient 7,48 g de produit attendu brut. F≅200 °C.

On recristallise ce produit brut dans l'acétate d'éthyle en le traitant au charbon actif. On recueille 4,19 g de produit attendu. F=204 °C. Ce produit est identique au produit obtenu à l'exemple 1.

Exemple 5

2-(1-chloroéthyl) 4-hydroxy N-(2-thiazolyl) 8-trifluorométhyl 3-quinoléïne carboxamide.

On opère de manière analogue à celle décrite à l'exemple 4, au départ de 1,5 g d'aminothiazole et de 1,041 g de 2-(1-chloroéthyl) 4-hydroxy 8-trifluorométhyl 3-quinoléïne carboxylate d'éthyle. Après addition d'acide chlorhydrique 1N, on extrait à l'acétate d'éthyle, lave la phase organique à l'eau, sèche et concentre à sec. On purifie le produit par chromatographie sur silice en éluant à l'acétate d'éthyle et obtient le produit attendu que l'on purifie dans l'éther. F≅190 °C. (Décomposition).

Le 2-(1-chloroéthyl) 4-hydroxy 8-trifluorométhyl 3-quinoléïne carboxylate d'éthyle a été préparé comme suit:

On mélange 9,3 g de 2-éthyl 4-hydroxy 8-trifluorométhyl 3-quinoléïne carboxylate d'éthyle, 200 $cm^3$ de tétrachlorure de carbone, 4,2 g de N-chloro succinimide et 0,45 g d'azobis isobutyronitrile, maintient sous agitation pendant 5 heures en chauffant à 65 °C maximum par l'intermédiaire d'une lampe, refroidit à 20 °C, filtre et concentre le filtrat à sec. On reprend le résidu au chlorure de

méthylène, lave à l'eau, sèche et évapore le solvant. On empâte le résidu à l'éther de pétrole, le sèche et obtient 9 g de produit attendu. F=104 °C.

Exemples 6 à 10

En opérant selon le procédé décrit à l'exemple 1 (Procédé A) ou selon le procédé décrit à l'exemple 2 (Procédé B), on a préparé au départ de l'acide 2-(dichlorométhyl) 4-hydroxy 8-trifluorométhyl 3-quinoléïne carboxylique et de son ester éthylique les produits indiqués ci-après.

Exemple 6

2-dichlorométhyl 4-hydroxy N-(4-phényl 2-thiazolyl) 8-trifluorométhyl 3-quinoléïne carboxamide (Procédé B). F=210 °C. (Décomposition).

Exemple 7

2-dichlorométhyl 4-hydroxy N-(5-méthyl 2-thiazolyl) 8-trifluorométhyl 3-quinoléïne carboxamide. (Procédé B). F=240–245 °C (Décomposition).

Exemple 8

2-dichlorométhyl 4-hydroxy N-(4,5-dihydro 2-thiényl) 8-trifluorométhyl 3-quinoléïne carboxamide (Procédé B). F≅205 °C. (Décomposition).

Exemple 9

2-dichlorométhyl 4-hydroxy N-(2-benzothiazolyl) 8-trifluorométhyl 3-quinoléïne carboxamide. (Procédé B). F≅265 °C. (Décomposition).

Exemple 10

2-dichlorométhyl 4-hydroxy N-méthyl N-(2-thiazolyl) 8-trifluorométhyl 3-quinoléïne carboxamide (Procédé A). F=228–230 °C.

Exemple 11

2-dichlorométhyl 4-hydroxy N-(3-méthyl 2-thiazolylidène) 8-trifluorométhyl 3-quinoléïne carboxamide (Procédé A). F=270–280 °C (Décomposition). Dans ce cas, le dérivé thiazolique de départ utilisé a été l'imine au lieu de l'amine.

En plus des produits mentionnés précédemment, les produits suivants peuvent également être obtenus selon l'invention:

–le 2-dichlorométhyl 4-hydroxy N-(5-chloro 2-thiazolyl) 8-trifluorométhyl 3-quinoléïne carboxamide. F=245 °C; (avec décomposition);

– le 2-dichlorométhyl 4-hydroxy N-(2-oxazolyl) 8-trifluorométhyl 3-quinoléïne carboxamide. F≅220 °C (avec décomposition);

– le 2-dichlorométhyl 4-hydroxy N-(1-méthyl 2-imidazolyl) 8-trifluorométhyl 3-quinoléïne carboxamide. F=240 °C (avec décomposition);

– le 2-dichlorométhyl 4-hydroxy N-(4-diméthylamino 3-méthyl 2-thiazolyl) 8-trifluorométhyl 3-quinoléïne carboxamide,

– le 2-chlorométhyl 4-hydroxy N-(2-thiazolyl) 8-trifluorométhyl 3-quinoléïne carboxamide. F=218 °C (Décomposition);

– le 2-chlorométhyl 4-hydroxy 8-trifluorométhyl 3-quinoléïne carboxylate d'éthyle utilisé au départ de la préparation du produit ci-dessus peut être préparé de la même manière que l'homologue 2-chloroéthyl, au départ du 2-méthyl 4-hydroxy 8-trifluorométhyl 3-quinoléïne carboxylate d'éthyle;

– le 2-(1-dichloroéthyl) 4-hydroxy N-(2-thiazolyl) 8-trifluorométhyl 3-quinoléïne carboxamide. F=220 °C (Décomposition);

– le 2-(1-dichloroéthyl) 4-hydroxy 8-trifluorométhyl 3-quinoléïne carboxylate d'éthyle utilisé au départ de la préparation du produit ci-dessus peut être préparé de la même manière que l'homologue 1-chloroéthyl en poursuivant la chloration.

Exemple 12

2-(dichlorométhyl) 4-hydroxy N-(2-thiazolyl) 8-trifluorométhyl 3-quinoléïne carboxamide.

Stade A: 2-dichlorométhyl 8-trifluorométhyl 4H-3,1-benzoxazin-4-one.

On chauffe progressivement sous agitation 17,44 g d'acide 2-amino 3-(trifluorométhyl) benzoïque préparé selon la procédé indiqué dans J. Med. Chem. 16 (2) 101, 6 (1973) et 34,63 g de chlorure de dichloroacétyle. Aux alentours de 50 °C, on observe une prise en masse, ainsi qu'un dégagement rapide et régulier d'HCl qui se poursuit pendant 1 heure. On observe alors vers 100 °C une fluidification du milieu réactionnel, une intensification du dégagement d'HCl et une élévation de la température jusqu'à 127 °C. On poursuit le chauffage jusqu'à cessation du dégagement d'HCl et l'on obtient une solution brune. On refroidit dans un bain d'eau glacée tout en agitant. On essore le précipité formé, empâte dans l'éther, puis dans le méthanol. On lave au méthanol. On obtient 23,82 g du produit recherché fondant à 179 °C.

Stade B: 2-(dichloroacétylamino) β-oxo-N-(2-thiazolyl) 3-trifluorométhyl benzène propanamide.

On ajoute entre −3° et −1 °C sous agitation et sous azote, 87,7 $cm^3$ d'une solution de n-butyllithium dans l'hexane titrant 1,1 moles par litre, dans une solution renfermant 8,65 g de N-(2-thiazolyl) acétamide et 304 $cm^3$ de tétrahydrofurane. On maintient encore 20 minutes sous agitation entre −3 et –1 °C. On refroidit à −75 °C, et l'on ajoute à cette température sous bonne agitation entre −73 et −75 °C, 9,073 g de 2-dichlorométhyl 8-trifluorométhyl 4H-3,1-benzoxazin-4-one en solution dans 95,5 cc de tétrahydrofurane. On agite à −75 °C pendant 2 heures 30 la solution brune obtenue. On verse sur un mélange d'eau, de glace et d'HCl. On décante et essore l'insoluble. On obtient 935 mg du produit recherché. F=223 °C. On extrait les phases aqueuses à l'acétate d'éthyle. On lave à l'eau, sèche et évapore à sec. On obtient ainsi 15,2 g du produit brut recherché. On y ajoute 100 cc de chlorure de méthylène, triture, essore l'insoluble, empâte dans 30 cc de chlorure de méthylène, essore, lave avec 10 cc de chlorure de méthylène. On obtient 7,47 g du produit recherché fondant à 223 °C.

Stade C: 2-(dichlorométhyl) 4-hydroxy N-(2-thiazolyl) 8-trifluorométhyl 3-quinoléïne carboxamide.

a) On ajoute une solution renfermant 8,81 g de 2-(dichloroacétylamino) β-oxo N-(2-thiazolyl) 3-(trifluorométhyl) benzène propanamide et 100 cc de diméthylformamide dans une suspension renfermant 1,06 g d'hydrure de sodium et 50 $cm^3$ de diméthylformamide. On maintient sous agitation pendant 4 heures à la température ambiante. On verse sur un mélange eau, glace, HCl. On essore le précipité formé, le lave à l'eau et le dissout dans 300 $cm^3$ de chlorure de méthylène. On lave les phases chlorométhyléniques à l'eau et les sèche sur sulfate de magnésium. On filtre et évapore à sec. On obtient 8,5 g du produit brut fondant à 175 °C. On triture le produit dans l'éthanol, essore et obtient 6,95 g d'un produit que l'on recristallise dans 215 $cm^3$ d'acétate d'éthyle en éliminant des traces d'insoluble. On obtient 5,015 g du produit recherché fondant à 204 °C.

b) On opère de manière analogue à celle décrite au Stade C a) ci-dessus, en utilisant un équivalent de carbonate de potassium au lieu de l'hydrure de sodium. On obtient le produit attendu. F = 204 °C.

Exemple 13

2-chlorométhyl 4-hydroxy N-(2-thiazolyl) 8-trifluorométhyl 3-quinoléïne carboxamide.

Stade A: 2-chlorométhyl 8-trifluorométhyl 4H-3,1-benzoxazin-3-one.

En opérant comme au Stade A de l'exemple 12, à partir d'acide 2-amino 3-trifluorométhyl benzoïque et de chlorure de chloracéthyle, on a obtenu le produit recherché fondant à 76≅78 °C.

Stade B: 2-(chloroacétylamino) β-oxo N-(2-thiazolyl) 3-trifluorométhyl benzène propanamide.

En opérant comme au Stade B de l'exemple 12, à partir du produit préparé au stade A du présent exemple et de N-(2-thiazolyl) acétamide, on obtient le produit recherché fondant à 192≅194 °C.

Stade C: 2-chlorométhyl 4-hydroxy N-(2-thiazolyl) 8-trifluorométhyl 3-quinoléïne carboxamide.

En opérant comme au Stade C de l'exemple 12, à partir du produit préparé au stade B du présent exemple en utilisant comme base la diméthylaminopyridine et comme solvant le tétrahydrofuranne, on obtient le produit recherché. F = 218 °C.

Exemple 14

2-(1-chloroéthyl)-4-hydroxy N-(2-thiazolyl) 8-trifluorométhyl 3-quinoléïne carboxamide.

Stade A: 2-(1-chloroéthyl) 8-trifluorométhyl 4H-3,1-benzoxazin-4-one.

En opérant comme au stade A de l'exemple 12, à partir du chlorure de 1-chloropropionyle et de l'acide 2-amino 3-trifluorométhyl benzoïque, on a obtenu le produit recherché. F = 112 °C.

Stade B: 2-(1-chloropropionylamino) β-oxo 3-trifluorométhyl N-(2-thiazolyl) benzène propanamide.

En opérant comme au stade B de l'exemple 12, à partir du produit obtenu au stade A ci-dessus et de N-(2-thiazolyl) acétamide, on a obtenu le produit recherché. F = 215 °C.

Stade C: 2-(1-chloroéthyl) 4-hydroxy N-(2-thiazolyl) 8-trifluorométhyl 3-quinoléïne carboxamide.

On opère de manière analogue à celle décrite au stade C de l'exemple 13, au départ du produit obtenu au stade B-ci-dessus et obtient le produit attendu. F = 190–192 °C.

Exemple 15

2-(1,1-dichloroéthyl) 4-hydroxy N-(2-thiazolyl) 8-trifluorométhyl 3-quinoléïne carboxamide.

Stade A: 2-(1,1-dichloroéthyl) 8-trifluorométhyl 4H-3,1-benzoxazin-4-one.

En opérant comme au stade A de l'exemple 12, à partir d'acide 2-amino 3-trifluorométhyl benzoïque et de chlorure de dichloropropionyle, on a obtenu le produit recherché. F = 120 °C.

Stade B: 2-(1,1-dichloropropionylamino) β-oxo N-(2-thiazolyl) 3-trifluorométhyl benzène propanamide.

En opérant comme au stade B de l'exemple 12, à partir du produit obtenu au stade A ci-dessus et de N-(2-thiazolyl) acétamide, on a obtenu le produit recherché. F = 136–138 °C.

Stade C: 2-(1,1-dichloroéthyl) 4-hydroxy N-(2-thiazolyl) 8-trifluorométhyl 3-quinoléïne carboxamide.

On opère de manière analogue à celle décrite au stade C de l'exemple 13, au départ du produit obtenu au stade B ci-dessus et obtient le produit attendu. F = 220 °C.

Exemple 16

2-(difluorométhyl) 4-hydroxy N-(2-thiazolyl) 8-trifluorométhyl 3-quinoléïne carboxamide.

Stade A: 2-(difluorométhyl) 8-trifluorométhyl 4H-3,1-benzoxazin-4-one.

On opère comme au stade A de l'exemple 12, à partir d'acide 2-amino 3-trifluorométhyl benzoïque et d'anhydride de l'acide difluoroacétique et obtient le produit attendu. F = 82 °C.

Stade B: 2-(difluoroacétylamino β-oxo 3-trifluorométhyl N-(2-thiazolyl) benzène propanamide.

On opère comme au stade B de l'exemple 12, à partir du produit obtenu au stade A ci-dessus et de N-(2-thiazolyl) acétamide, et obtient le produit attendu. F = 206 °C.

Stade C: 2-(difluorométhyl) 4-hydroxy N-(2-thiazolyl) 8-trifluorométhyl 3-quinoléïne carboxamide.

On opère de manière analogue à celle décrite au stade C de l'exemple 13, au départ du produit obtenu au stade B ci-dessus et obtient le produit attendu. F = 226 °C.

Exemple 17

2-(dichlorométhyl) 4-hydroxy N-(2-oxazolyl) 8-trifluorométhyl 3-quinoléïne carboxamide.

Stade A: 2-(dichloroacétylamino) β-oxo N-(2-oxazolyl) 3-trifluorométhyl benzène propanamide.

En opérant comme au stade B de l'exemple 12, à partir du produit préparé au stade A de l'exemple 12, et du N-(2-oxazolyl) acétamide, on obtient le produit recherché.

Stade B: 2-(dichlorométhyl) 4-hydroxy N-(2-oxazolyl) 8-trifluorométhyl 3-quinoléïne carboxamide.

On opère de manière analogue à celle décrite au stade C de l'exemple 13, au départ du produit obtenu au stade A ci-dessus et obtient le produit attendu. F=220–225 °C.

Exemple 18

2-dichlorométhyl 4-hydroxy N-(1-méthyl 2-imidazolyl) 8-trifluorométhyl 3-quinoléïne carboxamide.

Stade A: 2-(dichloroacétylamino) β-oxo N-(1-méthyl 2-imidazolyl) 3-trifluorométhyl benzène propanamide.

En opérant comme au stade B de l'exemple 12, à partir du produit préparé au stade A de l'exemple 12 et du N-1-méthyl 2-imidazolyl) acétamide, on obtient le produit recherché.

Stade B: 2-dichlorométhyl 4-hydroxy N-(1-méthyl 2-imidazolyl) 8-trifluorométhyl 3-quinoléïne carboxamide.

On opére de manière analogue à celle décrite au stade C de l'exemple 13, au départ du produit obtenu au stade A ci-dessus et obtient le produit attendu. F≅240 °C (Décomposition).

Exemples de compositions pharmaceutiques.

On a préparé des comprimés répondant à la formule suivante:

| | |
|---|---|
| Produit de l'exemple 1 | 50 mg |
| Excipient q.s. pour un comprimé terminé à | 350 mg |

(Détail de l'excipient: lactose, talc, amidon, stéarate de magnésium).

Etude pharmacologique du produit de l'exemple 1.

1°) Etude de l'activité analgésique:

Le test employé est basé sur le fait signalé par R. KOSTER et Coll., (Fed, Proc., 1959, 1B, 412) selon lequel l'injection intrapéritonéale d'acide acétique provoque, chez la souris, des mouvements répétés d'étirement et de torsion pouvant persister pendant plus de six heures. Les analgésiques préviennent ou diminuent ce syndrome qui peut être considéré comme l'extériorisation d'une douleur abdominale diffuse. On utilise une solution d'acide acétique à 1% dans l'eau. La dose déclenchant le syndrome est dans ses conditions de 0,01 $cm^3/g$, soit 100 mg/kg d'acide acétique.

Le produit étudié est administré par voie buccale une demi-heure avant l'injection d'acide acétique, les souris étant à jeun depuis la veille de l'expérience.

Les étirements sont observés et comptés pour chaque souris, pendant une période d'observation de quinze minutes commençant aussitôt après l'injection d'acide acétique.

Les résultats sont exprimés au moyen de la $DA_{50}$, c'est-à-dire la dose qui permet d'obtenir une diminution de 50% du nombre des étirements par rapport aux animaux témoins.

La $DA_{50}$ trouvée a été de 0,6 mg/kg.

2° Etude de l'activité anti-inflammatoire.

L'activité anti-inflammatoire a été déterminée sur le test de l'oédème plantaire provoqué par la carraghénine chez le rat.

On administre, à des rats mâles pesant de 130 à 150 g, 0,05 $cm^3$ d'une suspension stérile à 1% de carraghénine dans l'articulation tibio-tarsienne d'une patte postérieure.

Simultanément, on administre le produit à étudier dans une suspension de carboxyméthylcellulose à 0,25% et de Tween à 0,02% par voie orale.

Le volume de la patte est mesuré avant l'administration, puis deux heures, quatre heures, six heures, huit heures et vingt-quatre heures après.

L'intensité de l'inflammation est maxima quatre à six heures après l'injection de carraghénine. La différence du volume des pattes des animaux traités et des témoins met en évidence l'action anti-inflammatoire du médicament.

Le produit a été trouvé inactif à la dose de 50 mg/kg.

**Revendications pour les Etats contractants: BE, CH, DE, FR, GB, IT, LI, LU, NL, SE**

1. Les composés de formule (I)

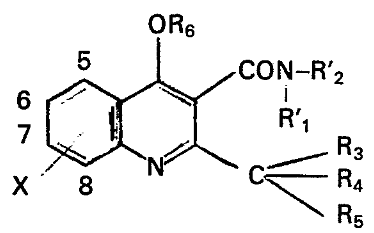

(I)

dans laquelle X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy $R_1'$ représente un atome d'hydrogène ou un radical alcoyle renfermant de 1 à 4 atomes de carbone, $R_2'$ représente un atome d'hydrogène ou un radical choisi parmi les radicaux thiazolyle, 4,5-dihydrothiazolyle, pyridinyle, oxazolyle, isoxazolyle, imidazolyle, pyrimidyle, tétrazolyle, thiényle, benzothiazolyle et phényle, éventuellement substitués par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyl et g) le radical nitro, ou $R_1'$ et $R_2'$ forment ensemble l'un quelconque des cycles saturés ou insaturés mentionnés ci-dessus pour $R_2'$, ledit cycle étant alors relié à l'atome d'azote par une double liaison, $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que $R_3$, $R_4$ et $R_5$ ne représentent pas en même temps chacun un atome de fluor et $R_6$ représente un atome d'hydrogène, un radical alcoyle renfermant de 1 à 8 atomes de carbone ou un radical acyle renfermant de 2 à 8 atomes de carbone, ainsi que leurs sels d'addition avec les acides et les bases.

2. Les composés selon la revendication 1, répondant à la formule (I)

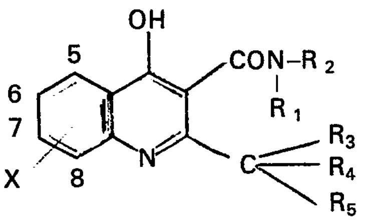

(I)

dans laquelle X est défini comme à la revendication 1, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle ou tétrazolyle, éventuellement substitué par un radical alcoyle renfermant de 1 à 4 atomes de carbone, et phényle, éventuellement substitué par au moins un radical choisi dans le groupe formé par les radicaux hydroxy, les radicaux alcoyles renfermant de 1 à 4 atomes de carbone, les radicaux aloxy renfermant de 1 à 4 atomes de carbone, le radical trifluorométhyle, le radical nitro et les atomes d'halogène et $R_3$, $R_4$ et $R_5$ sont définis comme à la revendication 1 ainsi que leurs sels d'addition avec les acides et les bases.

3. Les composés de formule I tels que définis à la revendication 2, pour lesquels $R_3$ représente un atome d'hydrogène, un radical alcoyle ou le même atome d'halogène que $R_5$, $R_4$ représente un atome d'hydrogène ou le même atome d'halogène que $R_5$, ainsi que leurs sels d'addition avec les acides et les bases.

4. Les composés de formule (I) tels que définis à la revendication 2 ou 3, pour lesquels X représente un radical trifluorométhyle, ainsi que leurs sels d'addition avec les acides et les bases.

5. Les composés de formule (I) tels que définis à l'une quelconque des revendications 2 à 4, pour lesquels $R_1$ représente un atome d'hydrogène ainsi que leurs sels d'addition avec les acides et les bases.

6. Les composés de formule (I) tels que définis à l'une quelconque des revendications 2 à 5, pour lesquels $R_3$ et $R_4$ identiques ou différents, représentent un atome d'hydrogène ou un atome de chlore, et $R_5$ représente un atome de chlore, ainsi que leurs sels d'addition avec les acides et les bases.

7. Les composés de formule (I) tels que définis à la revendication 6, pour lesquels le radical

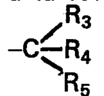

représente le radical $-CHCl_2$, ainsi que leurs sels d'addition avec les acides et les bases.

8. Les composés de formule (I) tels que définis à l'une quelconque des revendications 2 à 7, pour lesquels $R_2$ représente le radical thiazolyle, ainsi que leurs sels d'addition avec les acides et les bases.

9. Le 2-(dichlorométhyl) 4-hydroxy N-(2-thiazolyl) 8-trifluorométhyl 3-quinoléïne carboxamide, ainsi que ses sels d'addition avec les acides et les bases.

10. A titre de médicament, les composés de formule (I) tels que définis à l'une quelconque des revendications 1 à 8, ainsi que leurs sels d'addition avec les acides et les bases pharmaceutiquement acceptables.

11. A titre de médicament, le composé défini à la revendication 9, ainsi que ses sels d'addition avec les acides et les bases pharmaceutiquement acceptables.

12. Les compositions pharmaceutiques renfermant comme principe actif au moins un médicament défini à la revendication 10 ou 11.

13. Procédé de préparation des composés de formule (I) tel que défini à la revendication 1, caractérisé en ce que l'on soumet un composé de formule (II)

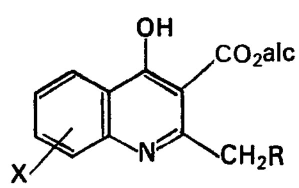

(II)

dans laquelle X conserve la même signification que dans la revendication 1, alc représente un radical alcoyle, renfermant de 1 à 8 atomes de carbone et R représente un atome d'hydrogène ou un radical alcoyle renfermant de 1 à 4 atomes de carbone à l'action d'un agent d'halogénation, puis le cas échéant, à l'action d'un second agent d'halogénation différent du premier, pour obtenir le composé de formule ($III_A$)

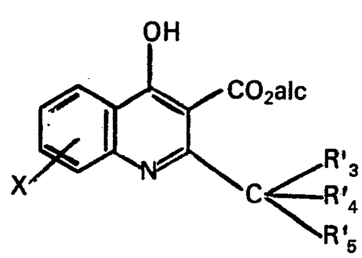

($III_A$)

dans laquelle $R_3'$, $R_4'$ et $R_5'$ conservent la signification donnée pour $R_3$, $R_4$ et $R_5$ précédemment, que l'on soumet, si désiré, à l'action d'un composé de formule $Hal_1M$, dans laquelle $Hal_1$, représente un atome d'halogène et M un atome de métal alcalin, pour obtenir le composé de formule ($III_B$)

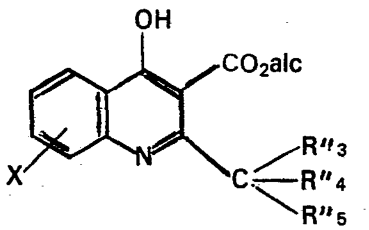

($III_B$)

dans laquelle $R_3''$ représente un atome d'hydrogène, un atome d'halogène $Hal_1$, un radical alcoyle renfermant de 1 à 4 atomes de carbone, $R_4''$ représente un atome d'hydrogène ou un atome d'halogène $Hal_1$ et $R_5''$ représente un atome d'halogène $Hal_1$, puis soumet le composé de formule ($III_A$) ou le composé de formule ($III_B$) à l'action d'un agent de saponification, pour obtenir un acide correspondant de formule (IV)

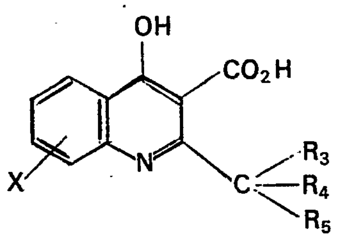

(IV)

dans laquelle $R_3$, $R_4$ et $R_5$ conservent leur signification précédente, que l'on transforme, si désiré en dérivé fonctionnel d'acide, puis soumet l'acide de formule (IV)ou un dérivé fonctionnel de cet acide, à l'action d'un composé de formule (V)

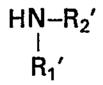

(V)

dans laquelle $R_1'$ et $R_2'$ conservent leur signification précédente pour obtenir le composé de formule ($I_A'$) correspondant

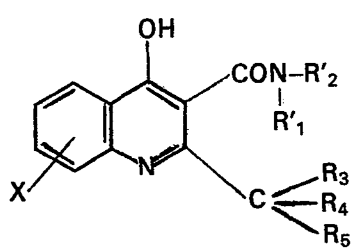

($I'_A$)

dans laquelle X, $R_1'$, $R_2'$, $R_3$, $R_4$ et $R_5$ sont définis comme précédemment, correspondant à un produit de formule (I') dans laquelle $R_6$ représente un atome d'hydrogène, produit de formule ($I_A'$) que l'on soumet, si désiré à l'action d'un agent d'éthérification ou d'estérification, pour obtenir un produit de formule ($I_B'$)

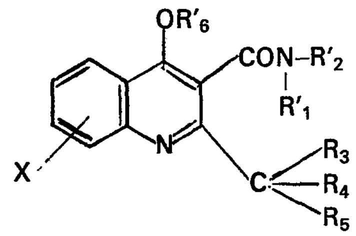

($I'_B$)

dans laquelle X, $R_1'$, $R_2'$, $R_3$, $R_4$ et $R_5$ sont définis comme précédemment, et $R_6'$ a les valeurs indiquées précédemment pour $R_6$, à l'exception d'hydrogène, produit de formule ($I_A'$) ou ($I_B'$) que, si désiré, l'on soumet à l'action d'un acide ou d'une base pour en former le sel.

14. Procédé selon la revendication 13, caractérisé en ce que l'on soumet un composé de formule ($II_a$)

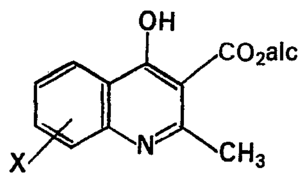

($II_a$)

dans laquelle X et alc conservent leur signification indiquée à la revendication 13, à l'action d'un agent d'halogénation, pour obtenir le composé de formule ($III_a$)

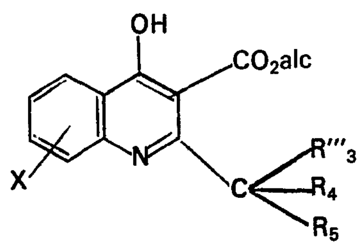

($III_a$)

dans laquelle $R_3'''$ et $R_4$ représentent, soit un atome d'hydrogène, soit le même atome d'halogène que $R_5$, produit ($III_a$), que l'on soumet à l'action d'un agent de saponification, pour obtenir un composé de formule ($IV_a$)

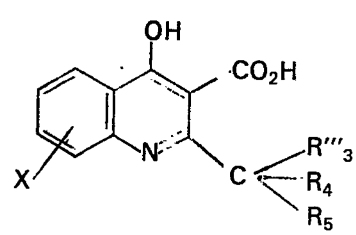

($IV_a$)

que l'on transforme, si désiré, en dérivé fonctionnel d'acide, puis soumet soit l'acide de formule ($IV_a$), soit un dérivé fonctionnel de cet acide, à l'action d'un composé de formule (V)

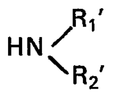
(V)

dans laquelle $R_1'$ et $R_2'$ conservent leur signification précédente, pour obtenir un composé de formule ($I_C'$)

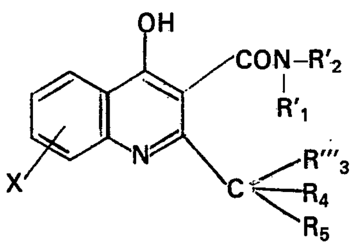
($I'_c$)

dans laquelle X, $R_1'$, $R_2'$, $R_3'''$ $R_4$ et $R_5$ sont définis comme précédemment, correspondant à un produit de formule (I') dans laquelle $R_6$ représente un atome d'hydrogène et $R_3$ a les valeurs de $R_3'''$, produit de formule ($I_C'$) que l'on soumet, si désiré, à l'action d'un agent d'éthérification ou d'estérification, pour obtenir un produit de formule ($I_D'$)

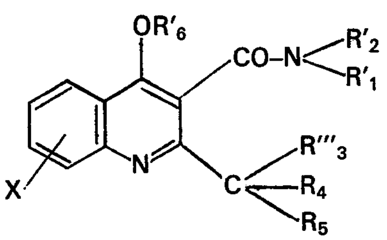
($I'_D$)

dans laquelle X, $R_1'$, $R_2'$, $R_3'''$, $R_4$ et $R_5$ sont définis comme précédemment et $R_6'$ a les valeurs indiquées précédemment pour $R_6$, à l'exception d'hydrogène, produit de formule ($I_C'$) ou ($I_D'$) que l'on soumet, si désiré, à l'action d'un acide ou d'une base pour en former le sel.

15. Procédé selon la revendication 13 ou 14, caractérisé en ce que l'agent d'halogénation utilisé est le N-chloro ou le N-bromosuccinimide.

16. Procédé selon la revendication 13 ou 14, caractérisé en ce que l'on soumet le composé de formule ($III_A$), ($III_B$) ou ($III_a$) à l'action d'un composé de formule (V)

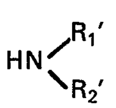
(V)

dans laquelle $R_1'$ et $R_2'$ sont définis comme à la revendication 13, en présence d'un trialcoylaluminium, pour obtenir un composé de formule ($I_A'$) ou ($I_C'$), telles que définies aux revendications 13 et 14, puis, si désiré, poursuit la synthèse comme décrit à la revendication 13 ou 14.

17. Procédé de préparation des composés de formule (I'), telle que définie à la revendication 1, caractérisé en ce que l'on soumet un composé de formule (VI)

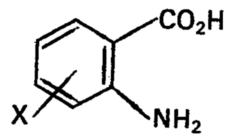
(VI)

dans laquelle X conserve sa signification donnée à la revendication 1, à l'action d'un acide de formule (VII)

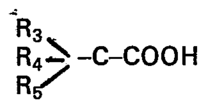
(VII)

dans laquelle $R_3$, $R_4$, $R_5$ conservent leurs significations données à la revendication 1 ou d'un dérivé fonctionnel de l'acide de formule (VII), pour obtenir un composé de formule (VIII)

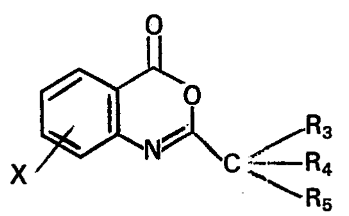
(VIII)

dans laquelle X conserve sa signification précédente, que l'on soumet à l'action d'un composé de formule (IX)

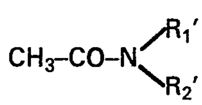
(IX)

dans laquelle $R_1'$ et $R_2'$ conservent leur significations données à la revendication 1, pour obtenir le composé de formule (X)

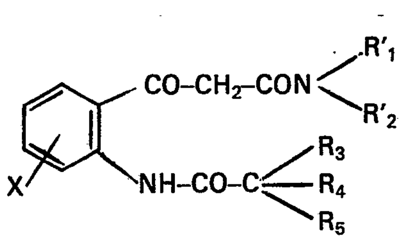
(X)

dans laquelle $R_1'$, $R_2'$, $R_3$, $R_4$ et $R_5$ conservent leurs significations précédentes, que l'on cyclise en présence d'un agent alcalin, pour obtenir un composé de formule ($I_A'$), telle que définie à la revendication 13, composé de formule ($I_A'$) que l'on soumet, si désiré, à l'action d'un agent d'éthérification ou d'estérification, pour obtenir un composé de formule ($I_B'$), telle que définie à la revendication 13, composé de formule ($I_A'$) ou ($I_B'$) que, si désiré, l'on soumet à l'action d'un acide ou d'une base pour en former le sel.

18. Procédé selon la revendication 17, caractérisé en ce que

– le dérivé fonctionnel de l'acide de formule (VII) est un halogénure ou un anhydride,

– la réaction entre le composé de formule (VIII) et le composé de formule (IX) a lieu en présence d'un organolithien ou d'un amidure de lithium.

19. A titre de produits industriels nouveaux, notamment à titre de produits intermédiaires, les composés de formules ($III_A$), ($III_B$) et (IV), tels que définis à la revendication 13.

20. A titre de produits industriels nouveaux, notamment à titre de produits intermédiaires, les composés de formules ($III_a$) et ($IV_a$), tels que définis à la revendication 14.

**Revendications pour l'Etat contractant: AT**

1. Procédé pour préparer les composés de formule (I')

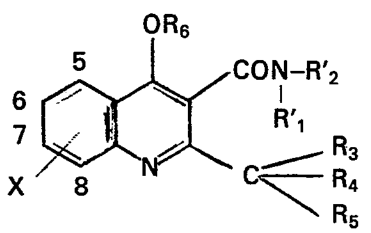
(I)

dans laquelle X en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyl, un radical trifluorométhyl thio ou un radical trifluorométhoxy, $R_1'$ représente un atome d'hydrogène ou un radical alcoyle renfermant de 1 à 4 atomes de carbone, $R_2'$ représente un atome d'hydrogène ou un radical choisi parmi les radicaux thiazolyle, 4,5-dihydrothiazolyle, pyridinyle, oxazolyle, isoxazolyle, imidazolyle, pyrimidyle, tétrazolyle, thiényle, benzothiazolyle et phényle, éventuellement substitués par un ou plusieurs radicaux choisis dans le groupe constitué par a) les halogènes, b) les radicaux alcoyles renfermant de 1 à 4 atomes de carbone, non substitués ou substitués par un radical amino, alcoylamino ou dialcoylamino dont les radicaux alcoyles renferment de 1 à 3 atomes de carbone, c) le radical phényle, d) les radicaux alcoxy renfermant de 1 à 4 atomes de carbone, e) le radical hydroxy, f) le radical trifluorométhyl et g) le radical nitro, ou $R_1'$ et $R_2'$ forment ensemble l'un quelconque des cycles saturés ou insaturés mentionnés ci-dessus pour $R_2'$, ledit cycle étant alors relié à l'atome d'azote par une double liaison, $R_3$ représente un atome d'hydrogène, un atome d'halogène, un radical alcoyle renfermant de 1 à 4 atomes de carbone, $R_4$ représente un atome d'hydrogène ou un atome d'halogène, $R_5$ représente un atome d'halogène, à la condition que $R_3$, $R_4$ et $R_5$ ne représentent pas en même temps chacun un atome de fluor et $R_6$ représente un atome d'hydrogène, un radical alcoyle renfermant de 1 à 8 atomes de carbone ou un radical acyle renfermant de 2 à 8 atomes de carbone, ainsi que leurs sels d'addition avec les acides et les bases, caractérisé en ce que soit l'on soumet un composé de formule (II)

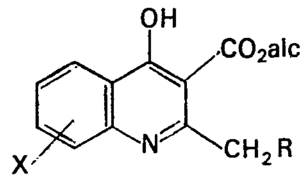
(II)

dans laquelle X conserve la même signification que précédemment, alc représente un radical alcoyle, renfermant de 1 à 8 atomes de carbone et R représente un atome d'hydrogène ou un radical alcoyle renfermant de 1 à 4 atomes de carbone, à l'action d'un agent d'halogénation, puis le cas échéant, à l'action d'un second agent d'halogénation différent du premier, pour obtenir le composé de formule ($III_A$)

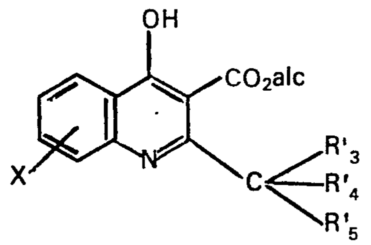
($III_A$)

dans laquelle $R_3'$, $R_4'$ et $R_5'$ conservent la signification donnée pour $R_3$, $R_4$ et $R_5$ précédemment, que l'on soumet, si désiré, à l'action d'un composé de formule $Hal_1M$, dans laquelle $Hal_1$ représente un atome d'halogène et M un atome de métal alcalin, pour obtenir le composé de formule ($III_B$)

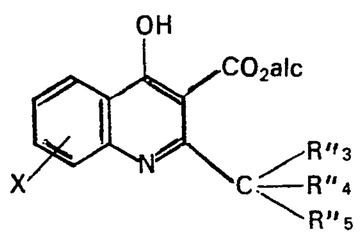
($III_B$)

dans laquelle $R_3''$ représente un atome d'hydrogène, un atome d'halogène $Hal_1$, un radical alcoyle renfermant de 1 à 4 atomes de carbone, $R_4''$ représente un atome d'hydrogène ou un atome d'halogène $Hal_1$ et $R_5''$ représente un atome d'halogène $Hal_1$, puis soumet le composé de formule ($III_A$) ou le composé de formule ($III_B$) à l'action

d'un agent de saponification, pour obtenir un acide correspondant de formule (IV)

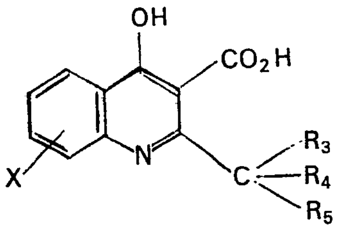

(IV)

dans laquelle $R_3$, $R_4$ et $R_5$ conservent leur signification précédente, que l'on transforme, si désiré, en dérivé fonctionnel d'acide, puis soumet l'acide de formule (IV) ou un dérivé fonctionnel de cet acide, à l'action d'un composé de formule (V)

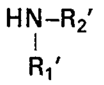

(V)

dans laquelle $R_1'$ et $R_2'$ conservent leur signification précédente pour obtenir le composé de formule ($I_A'$) correspondant

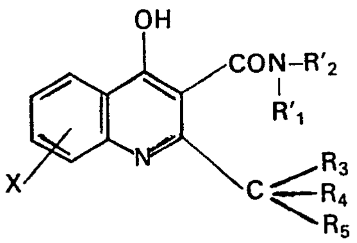

($I'_A$)

dans laquelle X, $R_1'$, $R_2'$ $R_3$, $R_4$ et $R_5$ sont définis comme précédemment, correspondant à un produit de formule (I') dans laquelle $R_6$ représente un atome d'hydrogène, produit de formule ($I_A'$) que l'on soumet, si désiré, à l'action d'un agent d'éthérification ou d'estérification, pour obtenir un produit de formule ($I_B'$)

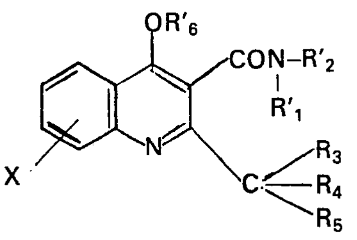

($I'_B$)

dans laquelle X, $R_1'$, $R_2'$, $R_3$, $R_4$ et $R_5$ sont définis comme précédemment, et $R_6'$ a les valeurs indiquées précédemment pour $R_6$, à l'exception d'hydrogène, produit de formule ($I_A'$) ou ($I_B'$) que, si désiré, l'on soumet à l'action d'un acide ou d'une base pour en former de sel, soit que l'on soumet un composé de formule (VI)

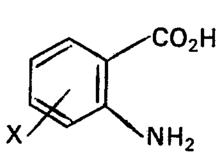

(VI)

dans laquelle X conserve sa signification donnée à la revendication 1, à l'action d'un acide de formule (VII)

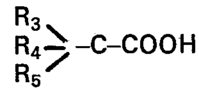

(VII)

dans laquelle $R_3$, $R_4$, $R_5$ conservent leurs significations données à la revendication 1 ou d'un dérivé fonctionnel de l'acide de formule (VII), pour obtenir un composé de formule (VIII)

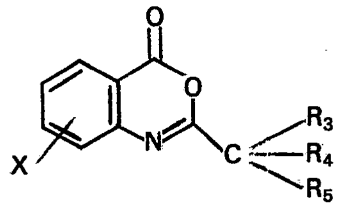

(VIII)

dans laquelle X conserve sa signification précédente, que l'on soumet à l'action d'un composé de formule (IX)

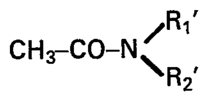

(IX)

dans laquelle $R_1'$ et $R_2'$ conservent leur significations données à la revendication 1, pour obtenir le composé de formule (X)

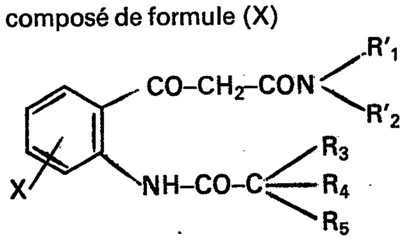

(X)

dans laquelle $R_1'$, $R_2'$, $R_3$, $R_4$ et $R_5$ conservent leurs significations précédentes, que l'on cyclise en présence d'un agent alcalin, pour obtenir un composé de formule ($I_A'$) telle que définie précédemment composé de formule ($I_A'$) que l'on soumet, si désiré à l'action d'un agent d'éthérification ou d'estérification, pour obtenir un composé de formule ($I_B'$), telle que définie précédemment, composé de ($I_A'$) ou ($I_B'$) que, si désiré, l'on soumet à l'action d'un acide ou d'une base pour en former le sel.

2. Procédé selon la revendication 1, pour préparer les composés de formule (I') telle que définie à la revendication 1, caractérisé en ce que l'on soumet un composé de formule II

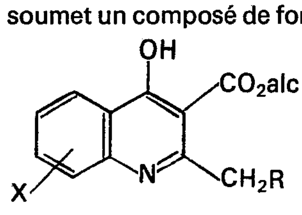

(II)

dans laquelle X conserve la même signification que précédemment, alc représente un radical alcoyle, renfermant de 1 à 8 atomes de carbone et R représente un atome d'hydrogène ou un radical alcoyle renfermant de 1 à 4 atomes de carbone, à l'action d'un agent d'halogénation, puis le cas échéant, à l'action d'un second agent d'halogénation différent du premier, pour obtenir le composé de formule ($III_A$)

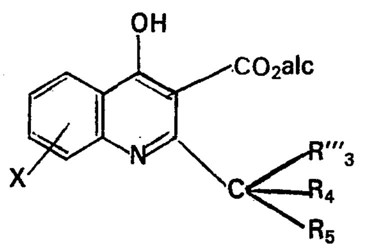

($III_A$)

dans laquelle $R_3'$, $R_4'$ et $R_5'$ conservent la signification donnée pour $R_3$, $R_4$ et $R_5$ précédemment, que l'on soumet, si désiré, à l'action d'un composé de formule $Hal_1M$, dans laquelle $Hal_1$ représente un atome d'halogène et M un atome de métal alcalin, pour obtenir le composé de formule ($III_B$)

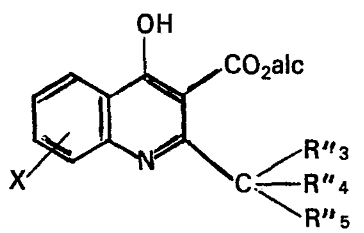

($III_B$)

dans laquelle $R_3''$ représente un atome d'hydrogène, un atome d'halogène $Hal_1$, un radical alcoyle renfermant de 1 à 4 atomes de carbone, $R_4''$ représente un atome d'hydrogène ou un atome d'halogène $Hal_1$ et $R_5''$ représente un atome halogène $Hal_1$, puis soumet le composé de formule ($III_A$) ou le composé de formule ($III_B$) à l'action d'un agent de saponification, pour obtenir un acide correspondant de formule (IV)

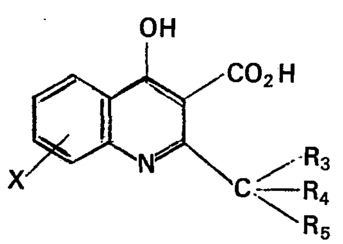

(IV)

dans laquelle $R_3$, $R_4$ et $R_5$ conservent leur signification précédente, que l'on transforme, si désiré, en dérivé fonctionnel d'acide, puis soumet l'acide de formule (IV) ou un dérivé fonctionnel de cet acide, à l'action d'un composé de formule (V)

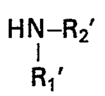

dans laquelle, $R_1'$ et $R_2'$ conservant leur signification précédente, pour obtenir le composé de formule ($I_A'$)

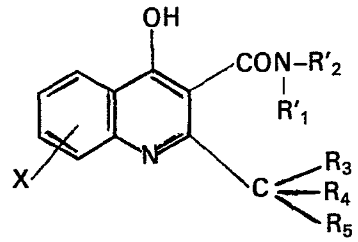

($I'_A$)

dans laquelle X, $R_1'$, $R_2'$, $R_3$, $R_4$ et $R_5$ sont définis comme précédemment, correspondant à un produit de formule (I') dans laquelle $R_6$ représente un atome d'hydrogéne, produit de formule ($I_A'$) que l'on soumet, si désiré, à l'action d'un agent d'éthérification ou d'estérification, pour obtenir un produit de formule ($I_B'$)

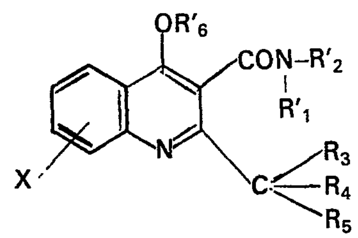

($I'_B$)

dans laquelle X, $R_1'$, $R_2'$, $R_3$, $R_4$ et $R_5$ sont définis comme précédemment et $R_6'$ a les valeurs indiquées précédemment pour $R_6$, à l'exception d'hydrogéne, produit de formule ($I_A'$) ou ($I_B'$) que, si désiré, l'on soumet à l'action d'un acide ou d'une base pour en former le sel.

3. Procédé selon la revendication 1, pour préparer les composés de formule (I') telle que définie à la revendication 1, caractérisé en ce que l'on soumet un composé de formule (VI)

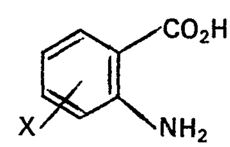

(VI)

dans laquelle S conserve sa signification précédemment, à l'action d'un acide de formule (VII)

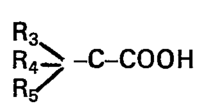

(VII)

dans laquelle $R_3$, $R_4$, $R_5$ conservent leurs significations données précédemment ou d'un dérivé fonctionnel de l'acide de formule (VII), pour obtenir un composé de formule (VIII)

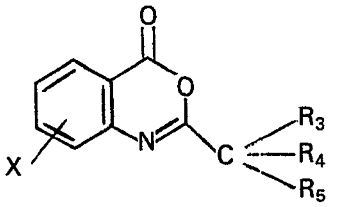

(VIII)

dans laquelle X conserve sa signification précédente, que l'on soumet à l'action d'un composé de formule (IX)

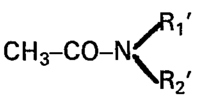

(IX)

dans laquelle $R_1'$ et $R_2'$ conserve leurs significations données précédemment, pour obtenir le composé de formule (X)

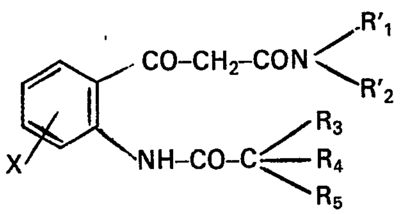

(X)

dans laquelle $R_1'$, $R_2'$, $R_3$, $R_4$ et $R_5$ conservent leurs significations précédentes, que l'on cyclise en présence d'un agent alcalin, pour obtenir un composé de formule $I_A'$, telle que définie précédemment, composé de formule ($I_A'$) que l'on soumet si désiré, à l'action d'un agent d'éthérification ou d'estérification, pour obtenir un composé de formule ($I_A'$) ou ($I_B'$) que, si désiré, l'on soumet à l'action d'un acide ou d'une base pour en former le sel.

4. Procédé selon la revendication 3, caractérisé en ce que le désiré fonctionnel de l'acide de formule (VII) est un halogène ou anhydride, la réaction entre le composé de formule (VIII) et le composé de formule (IX) a lieu en présence d'un organolithien ou d'un amidure de lithium.

5. Procédé selon la revendication 2, pour préparer les composés répondant à la formule (I)

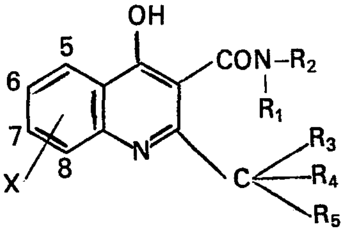

(I)

dans laquelle X est défini comme à la revendication 1, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle ou tétrazolyle, éventuellement substitué par un radical alcoyle renfermant de 1 à 4 atomes de carbone, et phényle, éventuellement substitué par un moins un radical choisi dans le groupe formé par les radicaux hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène et $R_3$, $R_4$ et $R_5$ sont définis comme à la revendication 1 ainsi que leurs sels d'addition avec les acides et les bases, caractérisé en ce que l'on soumet le composé de formule ($III_A$) ou le composé de formule ($III_B$) à l'action d'un agent de saponification, pour obtenir un acide correspondant de formule (IV)

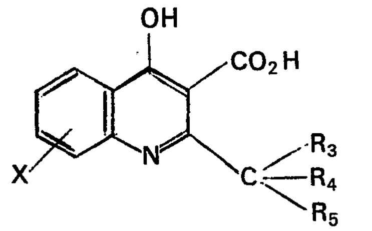

(IV)

dans laquelle $R_3$, $R_4$ et $R_5$ conservent leur signification précédente, que l'on transforme, si désiré, en dérivé fonctionnel d'acide, puis soumet l'acide de formule (IV) ou un dérivé fonctionnel de cet acide, à l'action d'un composé de formule (V')

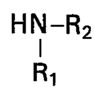

(V')

dans laquelle $R_1$ et $R_2$ sont définis comme précédemment, pour obtenir le composé de formule I, que, si désiré, l'on soumet à l'action d'un acide ou d'une base pour en former le sel.

6. Procédé selon la revendication 2, caractérisé en ce que l'on soumet un composé de formule ($II_a$)

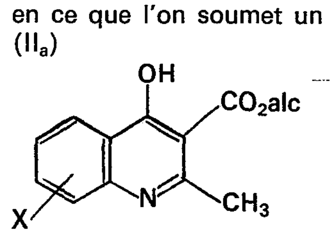

($II_a$)

dans laquelle X et alc conservent leur signification indiquée à la revendication 2, à l'action d'un agent d'halogénation, pour obtenir le composé de formule ($III_a$)

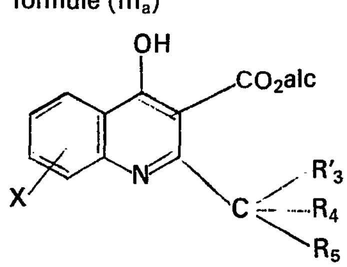

($III_a$)

dans laquelle $R_3'$ et $R_4$ représentent, soit un atome d'hydrogène, soit le même atome d'halogène que $R_5$, produit ($III_a$), que l'on soumet à l'action d'un agent de saponification, pour obtenir un composé de formule ($IV_a$)

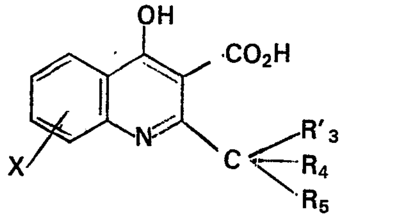
($IV_a$)

que l'on transforme, si désiré, en dérivé fonctionnel d'acide, puis soumet soit l'acide de formule ($IV_a$), soit un dérivé fonctionnel de cet acide, à l'action d'un composé de formule (V)

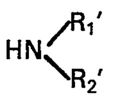
(V)

dans laquelle $R_1'$ et $R_2'$ conservent leur signification précédente, pour obtenir un composé de formule ($I_C'$)

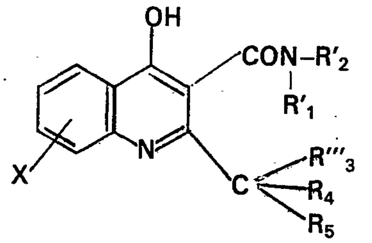
($I'_c$)

dans laquelle X, $R_1'$, $R_2'$, $R_3'''$, $R_4$ et $R_5$ sont définis comme précédemment, correspondant à un produit de formule (I') dans laquelle $R_6$ représente un atome d'hydrogène et $R_3$ a les valeurs de $R_3'''$, produit de formule ($I_C'$) que l'on soumet, si désiré, à l'action d'un agent d'éthérification ou d'estérification, pour obtenir un produit de formule ($I_D'$)

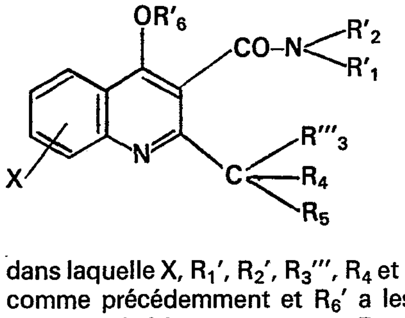
($I'_D$)

dans laquelle X, $R_1'$, $R_2'$, $R_3'''$, $R_4$ et $R_5$ sont définis comme précédemment et $R_6'$ a les valeurs indiquées précédemment pour $R_6$, à l'exception d'hydrogène, produit de formule ($I_C'$) ou ($I_D'$) que l'on soumet, si désiré, à l'action d'un acide ou d'une base pour en former le sel.

7. Procédé selon la revendication 6, pour préparer les composés répondant à la formule I, telle que définie à la revendication 4, ainsi que leurs sels, caractérisé en ce que l'on soumet un composé de formule ($II_a$)

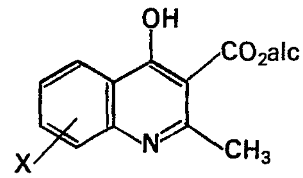
($II_a$)

dans laquelle X et alc conservent leur signification indiquée à la revendication 1, à l'action d'un agent d'halogénation, pour obtenir le composé de formule ($III_a$)

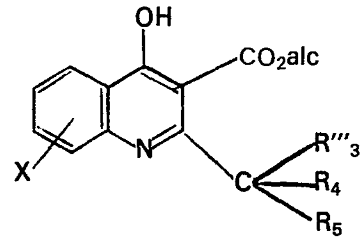
($III_a$)

dans laquelle $R_3'''$ et $R_4$ représentent, soit un atome d'hydrogène, soit le même atome d'halogène que $R_5$, produit ($III_a$), que l'on soumet à l'action d'un agent de saponification, pour obtenir un composé de formule ($IV_a$)

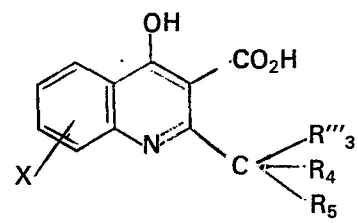
($IV_a$)

que l'on transformer, si désiré, en dérivé fonctionnel d'acide, puis soumet soit l'acide de formule ($IV_a$), soit un dérivé fonctionnel de cet acide, à l'action d'un composé de formule (V') dans laquelle $R_1$ et $R_2$ sont définis comme précédemment, pour obtenir le composé de formule I, que, si désiré, l'on soumet à l'action d'un acide ou d'une base pour en former le sel.

8. Procédé selon l'une quelconque des revendications 2, 5, 6 ou 7, caractérisé en ce que l'agent d'halogénation utilisé est le N-chloro ou le N-bromosuccinimide.

9. Procédé selon la revendication 2 ou 6, caractérisé en ce que l'on soumet le composé de formule ($III_A$), ($III_B$) ou ($III_a$) à l'action d'un composé de formule (V)

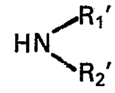
(V)

dans laquelle $R_1'$ et $R_2'$ sont définis à la revendication 2, en présence d'un trialcoylaluminium, pour obtenir un composé de formule ($I_A'$) ou ($I_C'$), telles que définies aux revendications 2 ou 6, puis, si désiré, poursuit la synthèse comme décrit à la revendication 2 ou 6.

10. Procédé selon l'une quelconque des revendications 1 à 9, caractérisé en ce que l'on utilise au départ un composé de formule (II), ($II_a$) ou (VI) dans lequel X représente un radical trifluorométhyle.

11. Procédé selon l'une quelconque des revendications 1 à 9, caractérisé en ce que l'on utilise au départ un composé de formule (V), (V') ou (IX) dans lequel $R_1'$ ou $R_1$ représente un atome d'hydrogène.

12. Procédé selon l'une quelconque des revendications 1 à 9, caractérisé en ce que l'on utilise au départ un composé de formule (V), (V') ou (IX) dans lequel $R_2'$ ou $R_2$ représente un radical thiazolyle.

**Patentansprüche für die Vertragsstaaten: BE, CH, DE, FR, GB, IT, LI, LU, NL und SE**

1. Verbindungen der Formel (I')

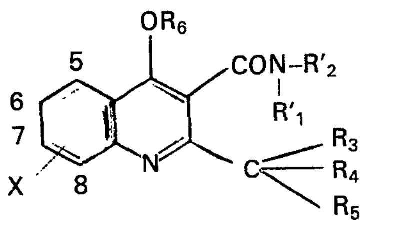

(I')

worin X in 5-, 6-, 7- oder 8-Stellung ein Wasserstoffatom, ein Halogenatom, einen linearen oder verzweigten Alkylrest mit 1 bis 5 Kohlenstoffatomen, einen linearen oder verzweigten Alkoxyrest mit 1 bis 4 Kohlenstoffatomen, einen Trifluormethylrest, einen Trifluormethylthiorest oder einen Trifluormethoxyrest bedeutet: $R_1'$ ein Wasserstoffatom oder einen Alkylrest mit 1 bis 4 Kohlenstoffatomen darstellt; $R_2'$ ein Wasserstoffatom oder einen Rest darstellt, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Thienyl-, Benzothiazolyl- und Phenylresten, gegebenenfalls substituiert durch einen oder mehrere Reste, ausgewählt unter der Gruppe, bestehend aus (a) den Halogenen, (b) den Alkylresten mit 1 bis 4 Kohlenstoffatomen, nichtsubstituiert oder substituiert durch einen Amino-, Alkylamino- oder Dialkylaminorest, deren Alkylreste 1 bis 3 Kohlenstoffatome enthalten, (c) dem Phenylrest, (d) den Alkoxyresten mit 1 bis 4 Kohlenstoffatomen, (e) dem Hydroxyrest, (f) dem Trifluormethylrest und (g) dem Nitrorest, oder $R_1'$ und $R_2'$ gemeinsam irgendeinen der vorstehend für $R_2'$ erwähnten, gesättigten oder ungesättigten Ringe bilden, wobei dieser Ring an das Stickstoffatom durch eine Doppelbindung gebunden ist; $R_3$ ein Wasserstoffatom, ein Halogenatom, einen Alkylrest mit 1 bis 4 Kohlenstoffatomen darstellt; $R_4$ ein Wasserstoffatom oder ein Halogenatom bedeutet; $R_5$ ein Halogenatom bedeutet, unter der Voraussetzung, dass $R_3$, $R_4$ und $R_5$ nicht gleichzeitig jeweils ein Fluoratom bedeuten; und $R_6$ ein Wasserstoffatom, einen Alkylrest mit 1 bis 8 Kohlenstoffatomen oder einen Acylrest mit 2 bis 8 Kohlenstoffatomen bedeutet; sowie deren Additionssalze mit Säuren und Basen.

2. Verbindungen gemäss Anspruch 1 der Formel (I)

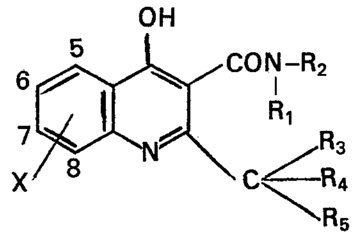

(I)

worin X wie in Anspruch 1 definiert ist; $R_1$ ein Wasserstoffatom oder einen Rest mit 1 bis 4 Kohlenstoffatomen bedeutet; $R_2$ einen Alkylrest, ausgewählt unter den Thiazolyl-, 4,5-Dihydrothiazolyl-, Pyridinyl-, Oxazolyl-, Isoxazolyl-, Imidazolyl-, Pyrimidyl- oder Tetrazolylresten, gegebenenfalls substituiert durch einen Alkylrest mit 1 bis 4 Kohlenstoffatomen, und Phenyl, gegebenenfalls substituiert durch zumindest einen Rest, ausgewählt aus der Gruppe, gebildet durch die Hydroxyreste, die Alkylreste mit 1 bis 4 Kohlenstoffatomen, die Alkoxyreste mit 1 bis 4 Kohlenstoffatomen, den Trifluormethylrest, den Nitrorest und die Halogenatome, bedeutet; und $R_3$, $R_4$ und $R_5$ wie in Anspruch 1 definiert sind; sowie deren Additionssalze mit Säuren und Basen.

3. Verbindungen der Formel (I) gemäss Anspruch 2, worin $R_3$ ein Wasserstoffatom, einen Alkylrest oder das gleiche Halogenatom wie $R_5$ bedeutet; $R_4$ ein Wasserstoffatom oder das gleiche Halogenatom wie $R_5$ bedeutet; sowie deren Additionssalze mit Säuren und Basen.

4. Verbindungen der Formel (I) gemäss Anspruch 2 oder 3, worin X einen Trifluormethylrest bedeutet, sowie deren Additionssalze mit Säuren und Basen.

5. Verbindungen der Formel (I) gemäss einem der Ansprüche 2 bis 4, worin $R_1$ ein Wasserstoffatom bedeutet, sowie deren Additionssalze mit Säuren und Basen.

6. Verbindungen der Formel (I) gemäss einem der Ansprüche 2 bis 5, worin $R_3$ und $R_4$ gleich oder verschieden sind und ein Wasserstoffatom oder ein Chloratom bedeuten und $R_5$ ein Chloratom bedeutet, sowie deren Additionssalze mit Säuren und Basen.

7. Verbindungen der Formel (I) gemäss Anspruch 6, worin der Rest 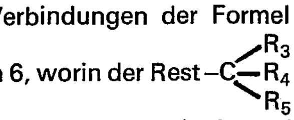 den Rest $-CHCl_2$ bedeutet, sowie deren Additionssalze mit Säuren und Basen.

8. Verbindungen der Formel (I) gemäss einem der Ansprüche 2 bis 7, worin $R_2$ den Thiazolylrest bedeutet, sowie deren Additionssalze mit Säuren und Basen.

9. 2-(Dichlormethyl)-4-hydroxy-N-(2-thiazolyl)-8-trifluormethyl-3-chinolin-carboxamid sowie dessen Additionssalze mit Säuren und Basen.

10. Als Arzneimittel die Verbindungen der Formel (I') gemäss einem der Ansprüche 1 bis 8 sowie deren pharmazeutisch verträgliche Additionssalze mit Säuren und Basen.

11. Als Arzneimittel die Verbindung gemäss Anspruch 9 sowie deren pharmazeutisch verträgliche Additionssalze mit Säuren und Basen.

12. Pharmazeutische Zusammensetzungen, enthaltend als Wirkstoff zumindest ein Arzneimittel gemäss Anspruch 10 oder 11.

13. Verfahren zur Herstellung der Verbindungen der Formel (I') gemäss Anspruch 1, dadurch gekennzeichnet, dass man eine Verbindung der Formel (II)

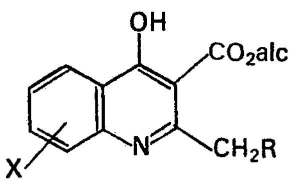

(II)

worin X die in Anspruch 1 angegebene Bedeutung besitzt, alc einen Alkylrest mit 1 bis 8 Kohlenstoffatomen bedeutet und R ein Wasserstoffatom oder einen Alkylrest mit 1 bis 4 Kohlenstoffatomen darstellt, der Einwirkung eines Halogenierungsmittels, dann gegebenenfalls der Einwirkung eines zweiten Halogenierungsmittels, das von dem ersten verschieden ist, unterzieht, um die Verbindung der Formel ($III_A$)

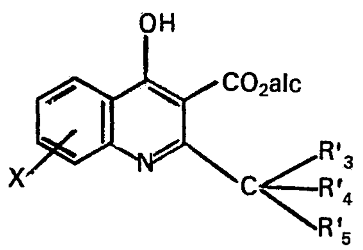

($III_A$)

zu erhalten, worin $R_3'$, $R_4'$ und $R_5'$ die vorstehend für $R_3$, $R_4$ und $R_5$ angegebene Bedeutung besitzen, die man gewünschtenfalls der Einwirkung einer Verbindung der Formel $Hal_1M$, worin $Hal_1$ ein Halogenatom bedeutet und M ein Alkalimetallatom darstellt, unterzieht, um die Verbindung der Formel ($III_B$)

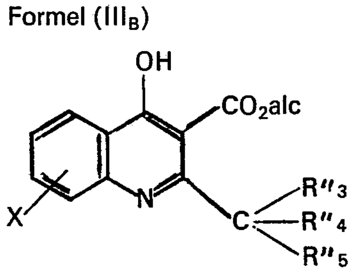

($III_B$)

zu erhalten, worin $R_3''$ ein Wasserstoffatom, ein Halogenatom $Hal_1$, einen Alkylrest mit 1 bis 4 Kohlenstoffatomen bedeutet, $R_4''$ ein Wasserstoffatom oder ein Halogenatom $Hal_1$ darstellt und $R_5''$ ein Halogenatom $Hal_1$ bedeutet, dann die Verbindung der Formel ($III_A$) oder die Verbindung der Formel ($III_B$) der Einwirkung eines Verseifungsmittels unterzieht, um eine entsprechende Säure der Formel (IV)

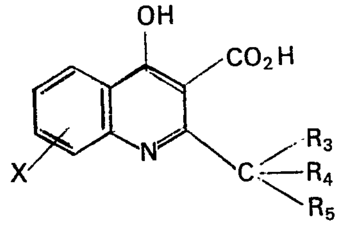

(IV)

zu erhalten, worin $R_3$, $R_4$ und $R_5$ die vorstehende Bedeutung besitzen, die man gewünschtenfalls in ein funktionelles Derivat der Säure überführt, danach die Säure der Formel (IV) oder ein funktionelles Derivat dieser Säure der Einwirkung einer Verbindung der Formel (V)

$HN-R_2'$
$\ \ |$
$\ R_1'$ (V)

unterzieht, worin $R_1'$ und $R_2'$ die vorstehend angegebene Bedeutung besitzen, um die entsprechende Verbindung der Formel ($I_A'$)

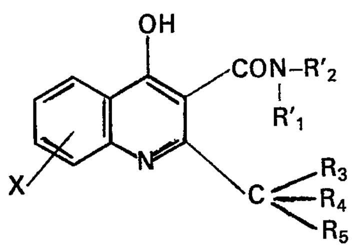

($I'_A$)

zu erhalten, worin X, $R_1'$, $R_2'$, $R_3$, $R_4$ und $R_5$ wie vorstehend definiert sind, entsprechend einem Produkt der Formel (I'), worin $R_6$ ein Wasserstoffatom bedeutet, welches Produkt der Formel ($I_A'$) man gewünschtenfalls der Einwirkung eines Veretherungs- oder Veresterungsmittels unterzieht, um ein Produkt der Formel ($I_B'$)

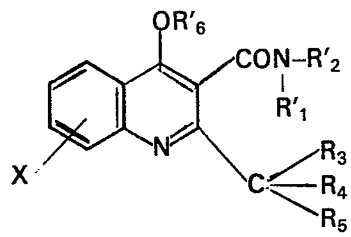

($I'_B$)

zu erhalten, worin X, $R_1'$, $R_2'$, $R_3$, $R_4$ und $R_5$ wie vorstehend definiert sind, und $R_6'$ die vorstehend für $R_6$ mit Ausnahme von Wasserstoff angegebenen Bedeutungen besitzt, welches Produkt der Formel ($I_A'$) oder ($I_B'$) man gewünschtenfalls der Einwirkung einer Säure oder einer Base unterzieht, um hieraus das Salz zu bilden.

14. Verfahren gemäss Anspruch 13, dadurch gekennzeichnet, dass man eine Verbindung der Formel ($II_a$)

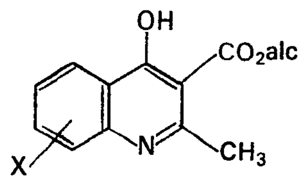

($II_a$)

worin X und alc die in Anspruch 13 angegebene Bedeutung besitzen, der Einwirkung eines Halogenierungsmittels unterzieht, um die Verbindung der Formel ($III_a$)

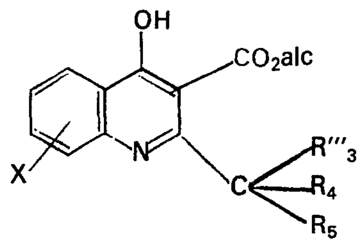

($III_a$)

zu erhalten, worin $R_3'''$ und $R_4$ entweder ein Wasserstoffatom oder das gleiche Halogenatom wie $R_5$ bedeuten, welches Produkt der Formel ($III_a$) man der Einwirkung eines Verseifungsmittels unterzieht, um eine Verbindung der Formel ($IV_a$)

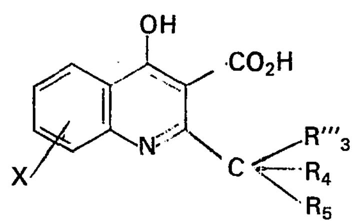

($IV_a$)

zu erhalten, die man gewünschtenfalls in ein funktionelles Derivat der Säure überführt, danach entweder die Säure der Formel ($IV_a$) oder ein funktionelles Derivat dieser Säure der Einwirkung einer Verbindung der Formel (V)

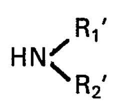

(V)

unterzieht, worin $R_1'$ und $R_2'$ die vorstehend angegebenen Bedeutungen besitzen, um eine Verbindung der Formel ($I_C'$)

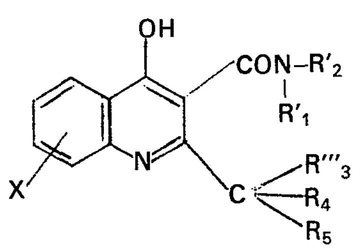

($I'_c$)

zu erhalten, worin X, $R_1'$, $R_2'$, $R_3'''$, $R_4$ und $R_5$ wie vorstehend definiert sind, entsprechend einem Produkt der Formel (I'), worin $R_6$ ein Wasserstoffatom bedeutet, und $R_3$ die Bedeutungen von $R_3'''$ besitzt, welches Produkt der Formel ($I_C'$) man gewünschtenfalls der Einwirkung eines Veretherungs- oder Veresterungsmittels unterzieht, um ein Produkt der Formel ($I_D'$)

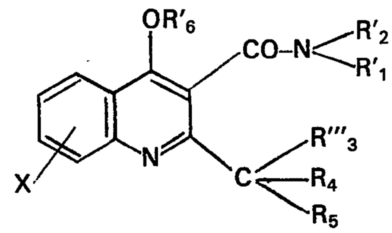

($I'_D$)

zu erhalten, worin X, $R_1'$, $R_2'$, $R_3'''$, $R_4$ und $R_5$ wie vorstehend definiert sind und $R_6'$ die vorstehend für $R_6$ angegebenen Bedeutungen mit Ausnahme von Wasserstoff besitzt, welches Produkt der Formel ($I_C'$) oder ($I_D'$) man gewünschtenfalls der Einwirkung einer Säure oder einer Base unterzieht, um hieraus das Salz zu bilden.

15. Verfahren gemäss Anspruch 13 oder 14, dadurch gekennzeichnet, dass das verwendete Halogenierungsmittel N-Chlor- oder N-Bromsuccinimid ist.

16. Verfahren gemäss Anspruch 13 oder 14, dadurch gekennzeichnet, dass man die Verbindung der Formel ($III_A$), ($III_B$) oder ($III_a$) der Einwirkung einer Verbindung der Formel (V)

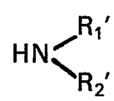

(V)

worin $R_1'$ und $R_2'$ wie in Anspruch 13 definiert sind, in Gegenwart eines Trialkylaluminiums unterzieht, um eine Verbindung der Formel ($I_A'$) oder ($I_C'$), wie in den Ansprüchen 13 und 14 definiert, zu erhalten, und danach gewünschtenfalls die Synthese, wie in Anspruch 13 oder 14, fortführt.

17. Verfahren zur Herstellung der Verbindungen der Formel (I') gemäss Anspruch 1, dadurch gekennzeichnet, dass man eine Verbindung der Formel (VI)

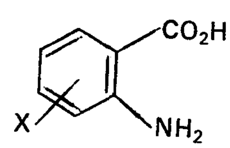

(VI)

worin X die in Anspruch 1 angegebene Bedeutung besitzt, der Einwirkung einer Säure der Formel (VII)

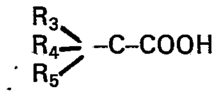
(VII)

unterzieht, worin $R_3$, $R_4$ und $R_5$ die in Anspruch 1 angegebenen Bedeutungen besitzen, oder derjenigen eines funktionellen Derivats der Säure der Formel (VII), um eine Verbindung der Formel (VIII)

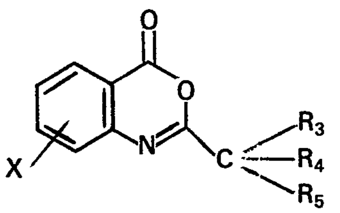
(VIII)

zu erhalten, worin X die vorstehende Bedeutung besitzt, die man der Einwirkung einer Verbindung der Formel (IX)

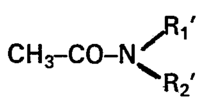
(IX)

unterzieht, worin $R_1'$ und $R_2'$ die in Anspruch 1 angegebenen Bedeutungen besitzen, um die Verbindung der Formel (X)

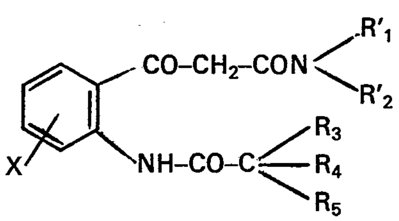
(X)

zu erhalten, worin $R_1'$, $R_2'$, $R_3$, $R_4$ und $R_5$ die vorstehenden Bedeutungen besitzen, die man in Gegenwart eines alkalischen Mittels cyclisiert, um eine Verbindung der Formel ($I_A'$), wie in Anspruch 13 definiert, zu erhalten, welche Verbindung der Formel ($I_A'$) man gewünschtenfalls der Einwirkung eines Verehterungsmittels oder Veresterungsmittels unterzieht, um eine Verbindung der Formel ($I_B'$), wie in Anspruch 13 definiert, zu erhalten, welche Verbindung der Formel ($I_A'$) oder ($I_B'$) man gewünschtenfalls der Einwirkung einer Säure oder einer Base unterzieht, um hieraus das Salz zu bilden.

18. Verfahren gemäss Anspruch 17, dadurch gekennzeichnet, dass

das funktionelle Derivat der Säure der Formel (VII) ein Halogenid oder ein Anhydrid ist,

die Umsetzung zwischen der Verbindung der Formel (VIII) und der Verbindung der Formel (IX) in Gegenwart einer Organolithiumverbindung oder eines Lithiumamids stattfindet.

19. Als neue, industrielle Produkte, insbesondere als Zwischenprodukte, die Verbindungen der Formeln ($III_A$), ($III_B$) und (IV), wie in Anspruch 13 definiert.

20. Als neue, industrielle Produkte, insbesondere als Zwischenprodukte, die Verbindungen der Formeln ($III_a$) und ($IV_a$), wie in Anspruch 14 definiert.

**Patentansprüche für den Vertragsstaat: AT**

1. Verfahren zur Herstellung von Verbindungen der Formel (I')

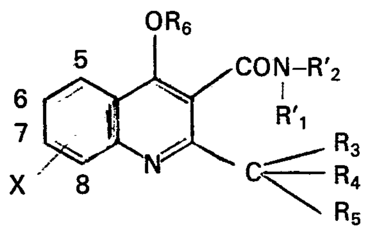
(I')

worin X in 5-, 6-, 7- oder 8-Stellung ein Wasserstoffatom, ein Halogenatom, einen linearen oder verzweigten Alkylrest mit 1 bis 5 Kohlenstoffatomen, einen linearen oder verzweigten Alkoxyrest mit 1 bis 4 Kohlenstoffatomen, einen Trifluormethylrest, einen Trifluormethylthiorest oder einen Trifluormethoxyrest bedeutet; $R_1'$ ein Wasserstoffatom oder einen Alkylrest mit 1 bis 4 Kohlenstoffatomen darstellt; $R_2'$ ein Wasserstoffatom oder einen Rest darstellt, ausgewählt unter den Thiazolyl-, 4,5-Dihydrothiazolyl-, Pyridinyl-, Oxazolyl-, Isoxazolyl-, Imidazolyl-, Pyrimidyl-, Tetrazolyl-, Thienyl-, Benzothiazolyl- und Phenylresten, gegebenenfalls substituiert durch einen oder mehrere Reste, ausgewählt unter der Gruppe, bestehend aus (a) den Halogenen, (b) den Alkylresten mit 1 bis 4 Kohlenstoffatomen, nichtsubstituiert oder substituiert durch einen Amino-, Alkylamino- oder Dialkylaminorest, deren Alkylreste 1 bis 3 Kohlenstoffatome enthalten (c) dem Phenylrest, (d) den Alkoxyresten mit 1 bis 4 Kohlenstoffatomen, (e) dem Hydroxyrest, (f) dem Trifluormethylrest und (g) dem Nitrorest, oder $R_1'$ und $R_2'$ gemeinsam irgendeinen der vorstehend für $R_2'$ erwähnten, gesättigten oder ungesättigten Ringe bilden, wobei dieser Ring an das Stickstoffatom durch eine Doppelbindung gebunden ist; $R_3$ ein Wasserstoffatom, ein Halogenatom, einen Alkylrest mit 1 bis 4 Kohlenstoffatomen darstellt; $R_4$ ein Wasserstoffatom oder ein Halogenatom bedeuten; $R_5$ ein Halogenatom bedeutet, unter der Voraussetzung, dass $R_3$, $R_4$ und $R_5$ nicht gleichzeitig jeweils ein Fluoratom bedeuten; und $R_6$ ein Wasserstoffatom, einen Alkylrest mit 1 bis 8 Kohlenstoffatomen oder einen Acylrest mit 2 bis 8 Kohlenstoffatomen bedeutet; sowie von deren Additionssalzen mit Säuren und Basen, dadurch gekennzeichnet, dass man eine Verbindung der Formel (II)

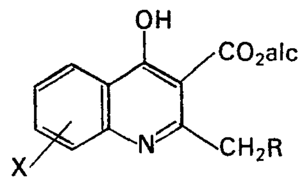

(II)

worin X die vorstehende Bedeutung besitzt, alc einen Alkylrest mit 1 bis 8 Kohlenstoffatomen bedeutet und R ein Wasserstoffatom oder einen Alkylrest mit 1 bis 4 Kohlenstoffatomen bedeutet, der Einwirkung eines Halogenierungsmittels und danach gegebenenfalls der Einwirkung eines zweiten Halogenierungsmittels, das von dem ersten verschieden ist, unterzieht, um die Verbindung der Formel ($III_A$)

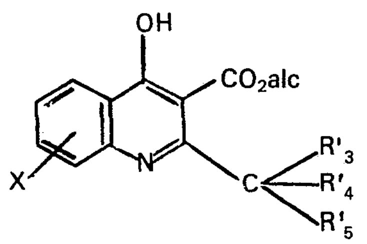

($III_A$)

zu erhalten, worin $R_3'$, $R_4'$ und $R_5'$ die für $R_3$, $R_4$ und $R_5$ vorstehend angegebene Bedeutung besitzen, die man gewünschtenfalls der Einwirkung einer Verbindung der Formel $Hal_1M$ unterzieht, worin $Hal_1$ ein Halogenatom bedeutet und M ein Alkalimetallatom darstellt, um die Verbindung der Formel ($III_B$)

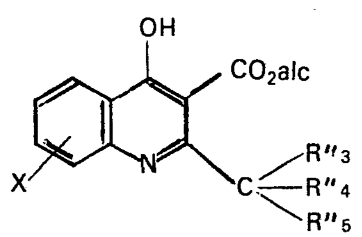

($III_B$)

zu erhalten, worin $R_3''$ ein Wasserstoffatom, ein Halogenatom $Hal_1$, einen Alkylrest mit 1 bis 4 Kohlenstoffatomen bedeutet, $R_4''$ ein Wasserstoffatom oder ein Halogenatom $Hal_1$ bedeutet und $R_5''$ ein Halogenatom $Hal_1$ bedeutet, danach die Verbindung der Formel ($III_A$) oder die Verbindung der Formel ($III_B$) der Einwirkung eines Verseifungsmittels unterzieht, um eine entsprechende Säure der Formel (IV)

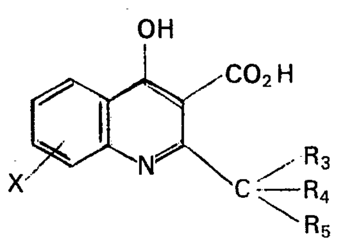

(IV)

zu erhalten, worin $R_3$, $R_4$ und $R_5$ die vorstehend angegebene Bedeutung besitzen, die man gewünschtenfalls in ein funktionelles Derivat der Säure überführt, danach die Säure der Formel (IV) oder ein funktionelles Derivat dieser Säure der Einwirkung einer Verbindung der Formel (V)

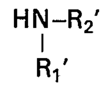

(V)

worin $R_1'$ und $R_2'$ die vorstehend angegebene Bedeutung besitzen, unterzieht, um die entsprechende Verbindung der Formel ($I_A'$)

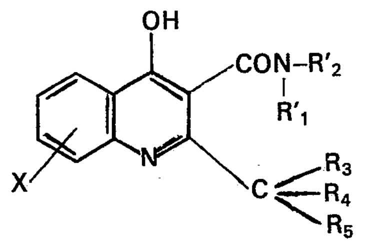

($I'_A$)

zu erhalten, worin X, $R_1'$, $R_2'$, $R_3$, $R_4$ und $R_5$ wie vorstehend definiert sind, entsprechend einem Produkt der Formel (I'), worin $R_6$ ein Wasserstoffatom bedeutet, welches Produkt der Formel ($I_A'$) man gewünschtenfalls der Einwirkung eines Veretherungs- oder Veresterungsmittels unterzieht, um ein Produkt der Formel ($I_B'$)

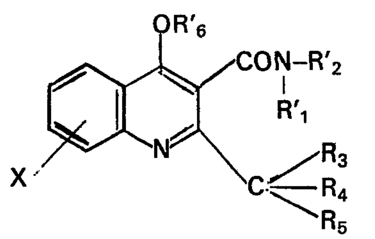

($I'_B$)

zu erhalten, worin X, $R_1'$, $R_2'$, $R_3$, $R_4$ und $R_5$ wie vorstehend definiert sind und $R_6'$ die vorstehend für $R_6$ angegebenen Bedeutungen mit Ausnahme von Wasserstoff besitzt, welches Produkt der Formel ($I_A'$) oder ($I_B'$) man gewünschtenfalls der Einwirkung einer Säure oder einer Base unterzieht, um hieraus das Salz zu bilden, oder dass man eine Verbindung der Formel (VI)

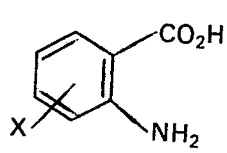

(VI)

worin X die vorstehend angegebene Bedeutung besitzt, der Einwirkung einer Säure der Formel (VII)

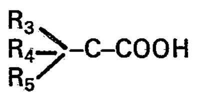

(VII)

unterzieht, worin $R_3$, $R_4$ und $R_5$ die vorstehend angegebenen Bedeutungen besitzen, oder derjenigen eines funktionellen Derivats der Säure der Formel (VII), um eine Verbindung der Formel (VIII)

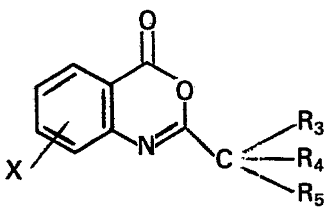

(VIII)

zu erhalten, worin X die vorstehende Bedeutung besitzt, die man der Einwirkung einer Verbindung der Formel (IX)

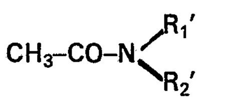

(IX)

unterzieht, worin $R_1'$ und $R_2'$ die vorstehend angegebenen Bedeutungen besitzen, um die Verbindung der Formel (X)

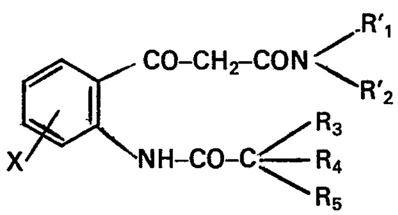

(X)

zu erhalten, worin $R_1'$, $R_2'$, $R_3$, $R_4$ und $R_5$ die vorstehenden Bedeutungen besitzen, die man in Gegenwart eines alkalischen Mittels cyclisiert, um eine Verbindung der Formel ($I_A'$), wie vorstehend definiert, zu erhalten, welche Verbindung der Formel ($I_A'$) man gewünschtenfalls der Einwirkung eines Veretherungs- oder Veresterungsmittels unterzieht, um eine Verbindung der Formel ($I_B'$), wie vorstehend definiert, zu erhalten, welche Verbindung der Formel ($I_A'$) oder ($I_B'$) man gewünschtenfalls der Einwirkung einer Säure oder einer Base unterzieht, um hieraus das Salz zu bilden.

2. Verfahren gemäss Anspruch 1 zur Herstellung der Verbindungen der Formel (I') gemäss Anspruch 1, dadurch gekennzeichnet, dass man eine Verbindung der Formel (II)

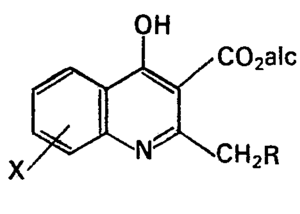

(II)

worin X die vorstehende Bedeutung besitzt, alc einen Alkylrest mit 1 bis 8 Kohlenstoffatomen bedeutet und R ein Wasserstoffatom oder einen Alkylrest mit 1 bis 4 Kohlenstoffatomen bedeutet, der Einwirkung eines Halogenierungsmittels und danach gegebenenfalls der Einwirkung eines zweiten Halogenierungsmittels, das von dem ersten verschieden ist, unterzieht, um die Verbindung der Formel ($III_A$)

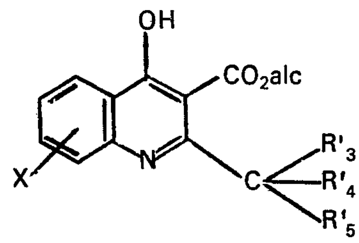

($III_A$)

zu erhalten, worin $R_3'$, $R_4'$ und $R_5'$ die für $R_3$, $R_4$ und $R_5$ vorstehend angegebene Bedeutung besitzen, die man gewünschtenfalls der Einwirkung einer Verbindung der Formel $Hal_1M$, worin $Hal_1$ ein Halogenatom bedeutet und M ein Alkalimetallatom darstellt, unterzieht, um die Verbindung der Formel ($III_B$)

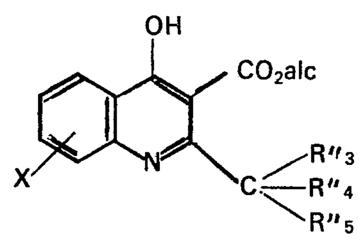

($III_B$)

zu erhalten, worin $R_3''$ ein Wasserstoffatom, ein Halogenatom $Hal_1$, einen Alkylrest mit 1 bis 4 Kohlenstoffatomen bedeutet, $R_4''$ ein Wasserstoffatom oder ein Halogenatom $Hal_1$ bedeutet und $R_5''$ ein Halogenatom $Hal_1$ bedeutet, danach die Verbindung der Formel ($III_A$) oder ($III_B$) der Einwirkung eines Verseifungsmittels unterzieht, um eine entsprechende Säure der Formel (IV)

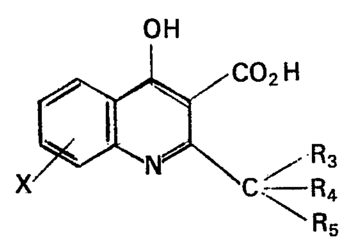

(IV)

zu erhalten, worin $R_3$, $R_4$ und $R_5$ die vorstehend angegebene Bedeutung besitzen, die man gewünschtenfalls in ein funktionelles Derivat der Säure überführt, danach die Säure der Formel (IV) oder ein funktionelles Derivat dieser Säure der Einwirkung einer Verbindung der Formel (V)

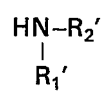

(V)

worin $R_1'$ und $R_2'$ die vorstehende Bedeutung besitzen, unterzieht, um die Verbindung der Formel ($I_A'$)

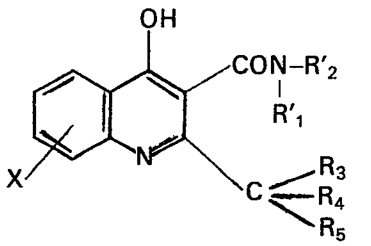

($I'_A$)

zu erhalten, worin X, $R_1'$, $R_2'$, $R_3$, $R_4$ und $R_5$ wie vorstehend definiert sind, entsprechend einem Produkt der Formel (I'), worin $R_6$ ein Wasserstoffatom bedeutet, welches Produkt der Formel ($I_A'$) man gewünschtenfalls der Einwirkung eines Veretherungs- oder Veresterungsmittels unterzieht, um ein Produkt der Formel ($I_B'$)

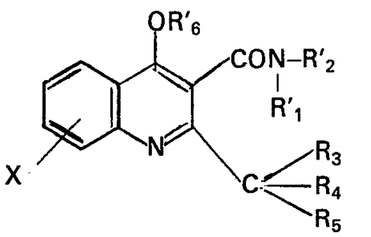

($I'_B$)

zu erhalten, worin X, $R_1'$, $R_2'$, $R_3$, $R_4$ und $R_5$ wie vorstehend definiert sind und $R_6'$ die vorstehend für $R_6$ angegebenen Bedeutungen mit Ausnahme von Wasserstoff besitzt, welches Produkt der Formel ($I_A'$) oder ($I_B'$) man gewünschtenfalls der Einwirkung einer Säure oder einer Base unterzieht, um hieraus das Salz zu bilden.

3. Verfahren gemäss Anspruch 1 zur Herstellung der Verbindungen der Formel (I') gemäss Anspruch 1, dadurch gekennzeichnet, dass man eine Verbindung der Formel (VI)

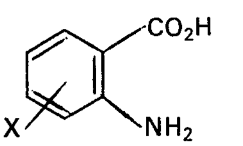

(VI)

worin X die vorstehend angegebene Bedeutung besitzt, der Einwirkung einer Säure der Formel (VII)

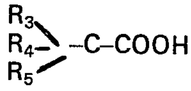

(VII)

unterzieht, worin $R_3$, $R_4$ und $R_5$ die vorstehend angegebenen Bedeutungen besitzen, oder derjenigen eines funktionellen Derivats der Säure der Formel (VII), um eine Verbindung der Formel (VIII)

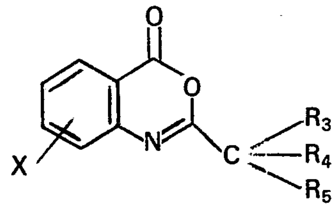

(VIII)

zu erhalten, worin X die vorstehende Bedeutung besitzt, die man der Einwirkung einer Verbindung der Formel (IX)

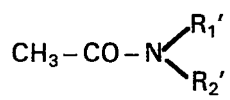

(IX)

unterzieht, worin $R_1'$ und $R_2'$ die vorstehend angegebene Bedeutung besitzen, um die Verbindung der Formel (X)

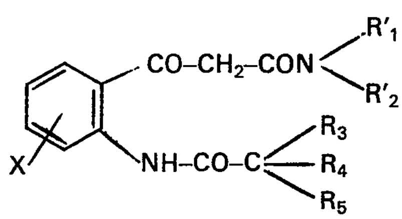

(X)

zu erhalten, worin $R_1'$, $R_2'$, $R_3$, $R_4$ und $R_5$ die vorstehenden Bedeutungen besitzen, die man in Gegenwart eines alkalischen Mittels cyclisiert, um eine Verbindung der Formel ($I_A'$), wie vorstehend definiert, zu erhalten, welche Verbindung der Formel ($I_A'$) man gewünschtenfalls der Einwirkung eines Veretherungs- oder Veresterungsmittels unterzieht, um eine Verbindung der Formel ($I_A'$) oder ($I_B'$) zu erhalten, die man gewünschtenfalls der Einwirkung einer Säure oder einer Base unterzieht, um hieraus das Salz zu bilden.

4. Verfahren gemäss Anspruch 3, dadurch gekennzeichnet, dass das gewünschte, funktionelle Derivat der Säure der Formel (VII) ein Halogenid oder Anhydrid ist, die Umsetzung zwischen der Verbindung der Formel (VIII) und der Verbindung der Formel (IX) in Gegenwart einer Organolithiumverbindung oder eines Lithiumamids stattfindet.

5. Verfahren gemäss Anspruch 2 zur Herstellung der Verbindungen der Formel (I)

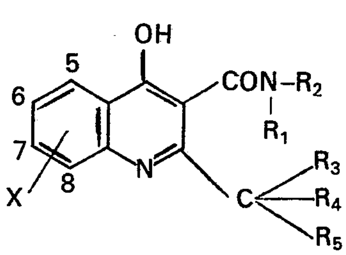

(I)

worin X wie in Anspruch 1 definiert ist, $R_1$ ein Wasserstoffatom oder einen Alkylrest mit 1 bis 4

Kohlenstoffatomen bedeutet, $R_2$ einen Rest, ausgewählt unter den Thiazolyl-, 4,5-Dihydrothiazolyl-, Pyridinyl-, Oxazolyl-, Isoxazolyl-, Imidazolyl-, Pyrimidyl- oder Tetrazolylresten, gegebenenfalls substituiert durch einen Alkylrest mit 1 bis 4 Kohlenstoffatomen, und Phenyl, gegebenenfalls substituiert durch zumindest einen Rest, ausqewählt aus der Gruppe, gebildet durch die Hydroxyreste, die Alkylreste mit 1 bis 4 Kohlenstoffatomen, die Alkoxyreste mit 1 bis 4 Kohlenstoffatomen, den Trifluormethylrest, den Nitrorest und die Halogenatome, bedeutet und $R_3$, $R_4$ und $R_5$ wie in Anspruch 1 definiert sind, sowie von deren Additionssalzen mit Säuren und Basen, dadurch gekennzeichnet, dass man die Verbindung der Formel ($III_A$) oder die Verbindung der Formel ($III_B$) der Einwirkung eines Verseifungsmittels unterzieht, um eine entsprechende Säure der Formel (IV)

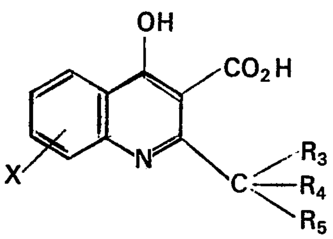

(IV)

zu erhalten, worin $R_3$, $R_4$ und $R_5$ die vorstehend angegebene Bedeutung besitzen, die man gewünschtenfalls in ein funktionelles Derivat der Säure überführt, danach die Säure der Formel (IV) oder ein funktionelles Derivat dieser Säure der Einwirkung einer Verbindung der Formel (V')

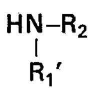

(V')

worin $R_1$ und $R_2$ wie vorstehend definiert sind, unterzieht, um die Verbindung der Formel (I) zu erhalten, die man gewünschtenfalls der Einwirkung einer Säure oder einer Base unterzieht, um hieraus das Salz zu bilden.

6. Verfahren gemäss Anspruch 2, dadurch gekennzeichnet, dass man eine Verbindung der Formel ($II_a$)

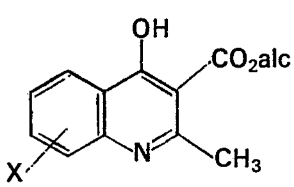

($II_a$)

worin X und alc die in Anspruch 2 angegebene Bedeutung besitzen, der Einwirkunq eines Halogenierungsmittels unterzieht, um die Verbindung der Formel ($III_a$)

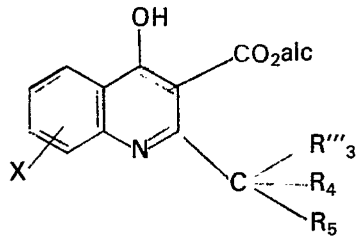

($III_a$)

zu erhalten, worin $R_3'$ und $R_4$ entweder ein Wasserstoffatom oder das gleiche Halogenatom wie 5 bedeuten, welches Produkt ($III_a$) man der Einwirkung eines Verseifungsmittels unterzieht, um eine Verbindung der Formel ($IV_a$)

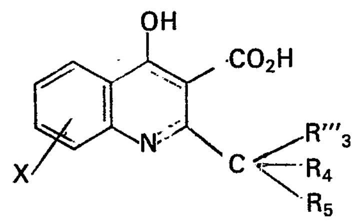

($IV_a$)

zu erhalten, die man gewünschtenfalls in ein funktionelles Derivat der Säure überführt, danach entweder die Säure der Formel ($IV_a$) oder ein funktionelles Derivat dieser Säure der Einwirkung einer Verbindung der Formel (V)

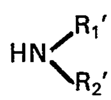

(V)

unterzieht, worin $R_1'$ und $R_2'$ die vorstehende Bedeutung besitzen, um eine Verbindung der Formel ($I_C'$)

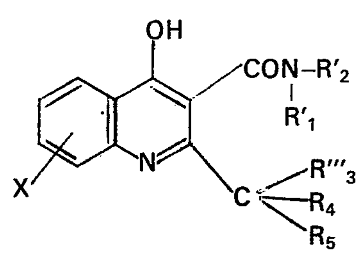

($I'_c$)

zu erhalten, worin X, $R_1'$, $R_2'$, $R_3'''$, $R_4$ und $R_5$ wie vorstehend definiert sind, entsprechend einem Produkt der Formel (I'), worin $R_6$ ein Wasserstoffatom bedeutet und $R_3$ die Bedeutung von $R_3'''$ besitzt, welches Produkt der Formel ($I_C'$) man gewünschtenfalls der Einwirkung eines Veretherungs- oder Veresterungsmittels unterzieht, um ein Produkt der Formel ($I_D'$)

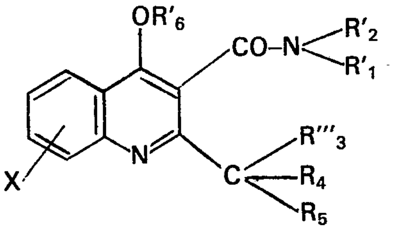

(I'D)

zu erhalten, worin X, $R_1'$, $R_2'$, $R_3'''$, $R_4$ und $R_5$ wie vorstehend definiert sind und $R_6'$ die vorstehend für $R_6$ angegebenen Bedeutungen mit Ausnahme von Wasserstoff besitzt, welches Produkt der Formel ($I_C'$) oder ($I_D'$) man gewünschtenfalls der Einwirkung einer Säure oder einer Base unterzieht, um hieraus das Salz zu bilden.

7. Verfahren gemäss Anspruch 6 zur Herstellung der Verbindungen der Formel (I) gemäss Anspruch 4 sowie von deren Salzen, dadurch gekennzeichnet, dass man eine Verbindung der Formel ($II_a$)

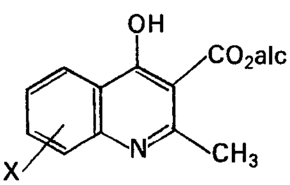

($II_a$)

worin X und alc die in Anspruch 1 angegebene Bedeutung besitzen, der Einwirkung eines Halogenierungsmittels unterzieht, um die Verbindung der Formel ($III_a$)

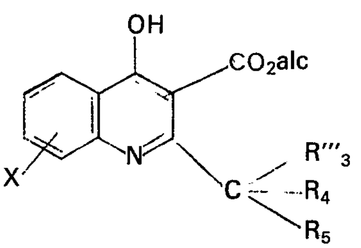

($III_a$)

zu erhalten, worin $R_3'''$ und $R_4$ entweder ein Wasserstoffatom oder das gleiche Halogenatom wie $R_5$ bedeuten, welches Produkt der Formel ($III_a$) man der Einwirkung eines Verseifungsmittels unterzieht, um eine Verbindung der Formel ($IV_a$)

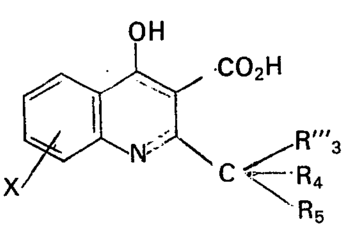

($IV_a$)

zu erhalten, die man gewünschtenfalls in ein funktionelles Derivat der Säure überführt, danach entweder die Säure der Formel ($IV_a$) oder ein funktionelles Derivat dieser Säure der Einwirkung der Verbindung der Formel (V') unterzieht, worin $R_1$ und $R_2$ wie vorstehend definiert sind, um die Verbindung der Formel (I) zu erhalten, die man gewünschtenfalls der Einwirkung einer Säure oder einer Base unterzieht, um hieraus das Salz zu bilden.

8. Verfahren gemäss einem der Ansprüche 2, 5, 6 oder 7, dadurch gekennzeichnet, dass das verwendete Halogenierungsmittel N-Chlor- oder N-Bromsuccinimid ist.

9. Verfahren gemäss Anspruch 2 oder 6, dadurch gekennzeichnet, dass man die Verbindung der Formel ($III_A$), ($III_B$) oder ($III_a$) der Einwirkung einer Verbindung der Formel (V)

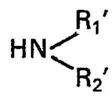

(V)

worin $R_1'$ und $R_2'$ wie in Anspruch 2 definiert sind, in Gegenwart eines Trialkylaluminiums unterzieht, um eine Verbindung der Formel ($I_A'$) oder ($I_C'$), wie in den Ansprüchen 2 oder 6 definiert, zu erhalten, und danach gewünschtenfalls die Synthese, wie in Anspruch 2 oder 6 beschrieben, fortsetzt.

10. Verfahren gemäss einem der Ansprüche 1 bis 9, dadurch gekennzeichnet, dass man von einer Verbindung der Formel (II), ($II_a$) oder (VI) ausgeht, worin X einen Trifluormethylrest bedeutet.

11. Verfahren gemäss einem der Ansprüche 1 bis 9, dadurch gekennzeichnet, dass man von einer Verbindung der Formel (V), (V') oder (IX) ausgeht, worin $R_1'$ oder $R_1$ ein Wasserstoffatom bedeuten.

12. Verfahren gemäss einem der Ansprüche 1 bis 9, dadurch gekennzeichnet, dass man von einer Verbindung der Formel (V), (V') oder (IX) ausgeht, worin $R_2'$ oder $R_2$ einen Thiazolylrest bedeuten.

**Claims for the contracting States: BE, CH, DE, FR, GB, IT, LI, LU, NL, SE**

1. The compounds with the formula (I'):

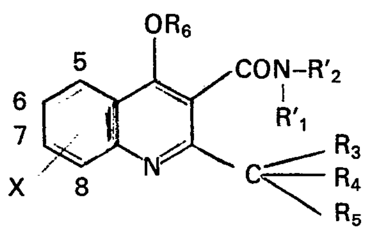

(I)

in which X in position 5, 6, 7 or 8 represents a hydrogen atom, a halogen atom, an alkyl radical, linear or branched, containing 1–5 carbon atoms, an alkoxy radical, linear or branched, containing 1–4 carbon atoms, a trifluoromethyl radical, a trifluoromethylthio radical or a trifluoromethoxy radical, $R_1'$ represents a hydrogen atom or an alkyl radical containing 1–4 carbon atoms, $R_2'$ repre-

sents a hydrogen atom or a radical chosen from the following radicals: thiazolyl, 4,5-dihydrothiazolyl, pyridinyl, oxazolyl, isoxazolyl, imidazolyl, pyrimidyl, tetrazolyl, thienyl, benzothiazolyl and phenyl, possibly substituted by one or more radicals chosen from the group constituted by a) the halogens, b) the alkyl radicals containing 1–4 carbon atoms, unsubstituted or substituted by an amino, alkylamino or dialkylamino radical, the alkyl radicals of which contain 1–3 carbon atoms, c) the phenyl radical, d) the alkoxy radicals containing 1–4 carbon atoms, e) the hydroxy radical, f) the trifluoromethyl radical and g) the nitro radical, or $R_1'$ and $R_2'$ together form any one of the saturated or unsaturated rings mentioned above for $R_2'$, the said ring then being linked to the nitrogen atom by a double bond, $R_3$ represents a hydrogenatom, a halogen atom, an alkyl radical containing 1–4 carbon atoms, $R_4$ represents a hydrogen atom or a halogen atom, $R_5$ represents a halogen atom, providing that $R_3$, $R_4$ and $R_5$ do not each represent a fluorine atom at the same time and $R_6$ represents a hydrogen atom, an alkyl radical containing 1–8 carbon atoms or an acyl radical containing 2–8 carbon atoms, as well as their salts of addition with acids and bases.

2. The compounds according to Claim 1, corresponding to formula (I):

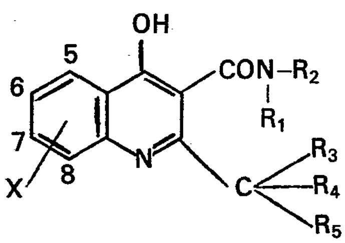

(I)

in which X is defined as in Claim 1, $R_1$ represents a hydrogen atom or an alkyl radical containing 1–4 carbon atoms, $R_2$ represents a radical chosen from the following radicals: thiazolyl, 4,5-dihydrothiazolyl, pyridinyl, oxazolyl, isoxazolyl, imidazolyl, pyrimidyl or tetrazolyl, possibly substituted by an alkyl radical containing 1–4 carbon atoms, and phenyl, possibly substituted by at least one radical chosen from the group formed by the hydroxy radicals, the alkyl radicals containing 1–4 carbon atoms, the alkoxy radicals containing 1–4 carbon atoms, the trifluoromethyl radical, the nitro radical and the halogen atoms and $R_3$, $R_4$ and $R_5$ are defined as in Claim 1, as well as their salts of addition with acids and bases.

3. The compounds with the formula (I) as defined in Claim 2, for which $R_3$ represents a hydrogen atom, an alkyl radical or the same halogen atom as $R_5$, $R_4$ represents a hydrogen atom or the same halogen atom as $R_5$, as well as their salts of addition with acids and bases.

4. The compounds with the formula (I) as defined in Claim 2 or 3, for which X represents a trifluoromethyl radical, as well as their salts of addition with acids and bases.

5. The compounds with the formula (I) as defined in any one of Claims 2–4, for which $R_1$ represents a hydrogen atom, as well as their salts of addition with acids and bases.

6. The compounds with the formula (I) as defined in any one of Claims 2–5, for which each of $R_3$ and $R_4$, identical or different, represents a hydrogen atom or a chlorine atom, and $R_5$ represents a chlorine atom, as well as their salts of addition with acids and bases.

7. The compounds with the formula (I) as defined in Claim 6, for which the radical 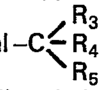 represents the radical $-CHCl_2$, as well as their salts of addition with acids and bases.

8. The compounds with the formula (I) as defined in any one of Claims 2–7, for which $R_2$ represents the thiazolyl radical, as well as their salts of addition with acids and bases.

9. 2-(dichloromethyl)-4-hydroxy-N-(2-thiazolyl)-8-trifluoromethyl-3-quinoline carboxamide, as well as its salts of addition with acids and bases.

10. As a medicament, the compounds with the formula (I') as defined in any one of Claims 1–8, as well as their salts of addition with pharmaceutically acceptable acids and bases.

11. As a medicament, the compound defined in Claim 9, as well as its salts of addition with pharmaceutically acceptable salts and bases.

12. The pharmaceutical compositions containing as active principle at least one medicament defined in Claim 10 or 11.

13. Preparation process for the compounds with the formula (I') as defined in Claim 1, characterized in that a compound with the formula (II):

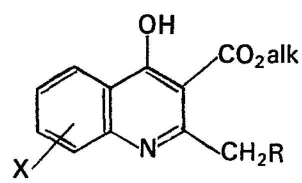

(II)

in which X retains the same significance as in Claim 1, alk represents an alkyl radical containing 1–8 carbon atoms and R represents a hydrogen atom or an alkyl radical containing 1–4 carbon atoms, is submitted to the action of a halogenation agent, then, if applicable, to the action of a second halogenation agent, different from the first, so as to obtain the compound with the formula ($III_A$):

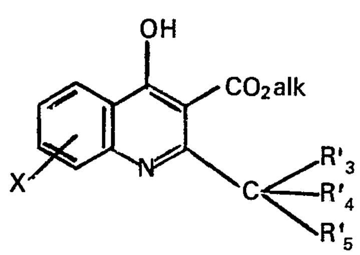

($III_A$)

in which $R_3'$, $R_4'$ and $R_5'$ retain the significance given previously for $R_3$, $R_4$ and $R_5$, which, if desired, is submitted to the action of a compound with the formula $Hal_1M$, in which $Hal_1$ represents a halogen atom and M represents an alkali metal atom, so as to obtain a compound with the formula ($III_B$):

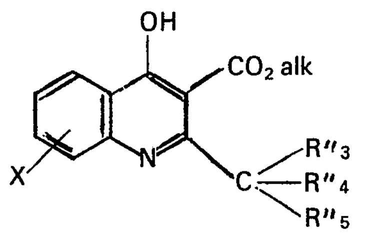

($III_B$)

in which $R_3''$ represents a hydrogen atom, a $Hal_1$ halogen atom, an alkyl radical containing 1–4 carbon atoms, $R_4''$ represents a hydrogen atom or a $Hal_1$ halogen atom, and $R_5''$ represents a $Hal_1$ halogen atom, then the compound with the formula ($III_A$) or the compound with the formula ($III_B$) is submitted to the action of a saponification agent, so as to obtain a corresponding acid with the formula (IV):

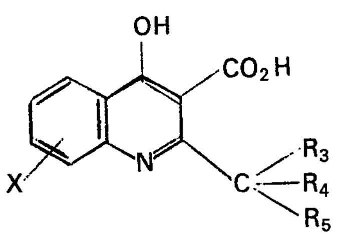

(IV)

in which $R_3$, $R_4$ and $R_5$ retain their preceding significance, which, if desired, is transformed into a functional derivative of the acid, then the acid with the formula (IV) or a functional derivative of this acid is submitted to the action of a compound with the formula (V):

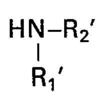

(V)

in which $R_1'$ and $R_2'$ retain their preceding significance, so as to obtain the corresponding compound with the formula ($I_A'$):

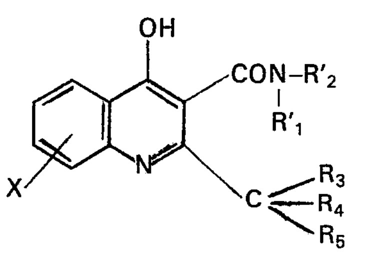

($I'_A$)

in which X, $R_1'$, $R_2'$, $R_3$, $R_4$ and $R_5$ are defined as previously, corresponding to a product with the formula (I') in which $R_6$ represents a hydrogen atom, which product with the formula ($I_A'$) is submitted, if desired, to the action of an etherification or esterification agent, so as to obtain a product with the formula ($I_B'$):

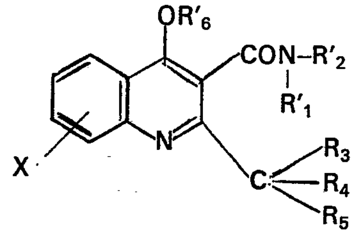

($I'_B$)

in which X, $R_1'$, $R_2'$, $R_3$, $R_4$ and $R_5$ are defined as previously and $R_6'$ has the previously indicated values for $R_6$, with the exception of hydrogen, which product with the formula ($I_A'$) or ($I_B'$) is submitted, if desired, to the action of an acid or a base so as to form its salt.

14. Process according to Claim 13, characterized in that a compound with the formula ($II_a$):

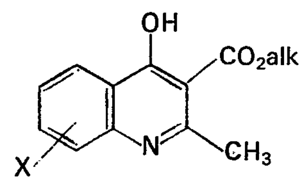

($II_a$)

in which X and alk retain their significance indicated in Claim 13, is submitted to the action of a halogenation agent, so as to obtain the compound with the formula ($III_a$):

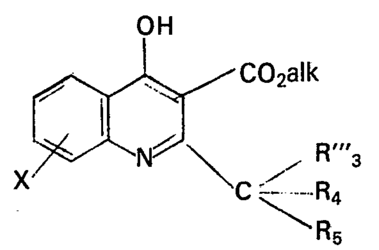

($III_a$)

in which $R_3'''$ and $R_4$ each represent either a hydrogen atom, or the same halogen atom as $R_5$, which product with the formula ($III_a$) is submitted to the action of a saponification agent, so as to obtain a compound with the formula ($IV_a$):

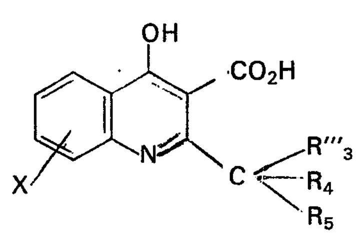

($IV_a$)

which, if desired, is transformed into a functional derivative of an acid then either the acid with the formula ($IV_a$), or a functional derivative of this

acid, is submitted to the action of a compound with the formula (V):

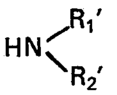

(V)

in which $R_1'$ and $R_2'$ retain their preceding significance, so as to obtain a compound with the formula ($I_C'$):

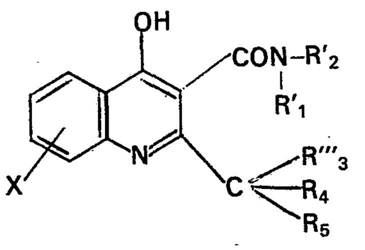

($I'_c$)

in which X, $R_1'$, $R_2'$, $R_3'''$, $R_4$ and $R_5$ are defined as previously, corresponding to a product with the formula (I') in which $R_6$ represents a hydrogen atom and $R_3$ has the values of $R_3'''$, which product with the formula ($I_C'$) is submitted, if desired, to the action of an etherification agent or an esterification agent, so as to obtain a product with the formula ($I_D'$):

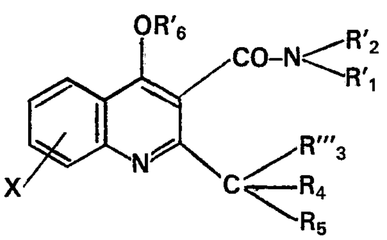

($I'_D$)

in which X, $R_1'$, $R_2'$, $R_3'''$, $R_4$ and $R_5$ are defined as previously and $R_6'$ has the values previously indicated for $R_6$, with the exception of hydrogen, which product with the formula ($I_C'$) or ($I_D'$) is submitted, if desired, to the action of an acid or a base so as to form its salt.

15. Process according to Claim 13 or 14, characterized in that the halogenation agent utilized is N-chloro or N-bromosuccinimide.

16. Process according to claim 13 or 14, characterized in that the compound with the formula ($III_A$), ($III_B$) or ($III_a$) is submitted to the action of a compound with the formula (V):

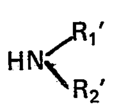

(V)

in which $R_1'$ and $R_2'$ are defined as in Claim 13, in the presence of trialkylaluminium, so as to obtain a compound with the formula ($I_A'$) or ($I_C'$), as defined in Claims 13 and 14, then, if desired, the synthesis is continued as described in Claim 13 or 14.

17. Preparation process for the compounds with the formula (I'), as defined in Claim 1, characterized in that a compound with the formula (VI):

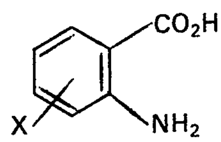

(VI)

in which X retains its significance given in Claim 1, is submitted to the action of an acid with the formula (VII):

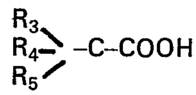

(VII)

in which $R_3$, $R_4$, $R_5$ retain their significances given in Claim 1 or a functional derivative of the acid with the formula (VII), so as to obtain a compound with the formula (VIII):

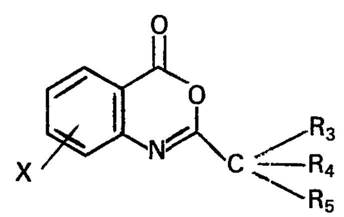

(VIII)

in which X retains its preceding significance, which is submitted to the action of a compound with the formula (IX):

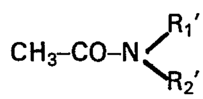

(IX)

in which $R_1'$ and $R_2'$ retain their significances given in Claim 1, so as to obtain the compound with the formula (X):

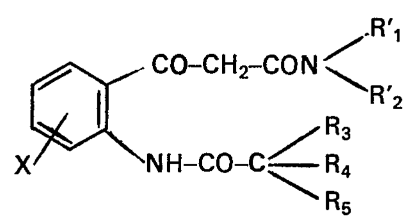

(X)

in which $R_1'$, $R_2'$, $R_3$, $R_4$ and $R_5$ retain their preceding significances, which is cyclized in the presence of an alkaline agent, so as to obtain a compound with the formula ($I_A'$), as defined in Claim 13, which compound with the formula ($I_A'$) is submitted, if desired, to the action of an etherification agent or an esterification agent, so as to obtain a compound with the formula ($I_B'$), as defined in Claim 13, which compound with the for-

mula ($I_A'$) or ($I_B'$), if desired, is submitted to the action of an acid or a base so as to form its salt.

18. Process according to Claim 17, characterized in that

– the functional derivative of the acid with the formula (VII) is a halide or an anhydride,

– the reaction between the compound with the formula (VIII) and the compound with the formula (IX) takes place in the presence of a organolithium compound or a lithium amide.

19. As new industrial products, notably as intermediate products, the compounds with the formula ($III_A$), ($III_B$) and (IV), as defined in Claim 13.

20. As new industrial products, notably as intermediate products, the compounds with the formulae ($III_a$) and ($IV_a$), as defined in Claim 14.

**Claims for the contracting State: AT**

1. Process for preparing the compounds with the formula (I'):

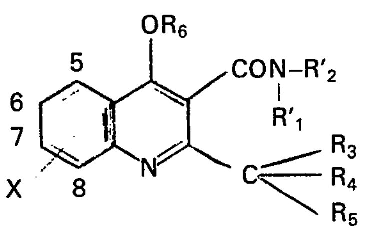

(I)

in which X in position 5, 6, 7 or 8 represents a hydrogen atom, a halogen atom, an alkyl radical, linear or branched, containing 1–5 carbon atoms, an alkoxy radical, linear or branched, containing 1–4 carbon atoms, a trifluoromethyl radical, a trifluoromethylthio radical, or a trifluoromethoxy radical, $R_1'$ represents a hydrogen atom or an alkyl radical containing 1–4 carbon atoms, $R_2'$ represents a hydrogen atom or a radical chosen from the following radicals: thiazolyl, 4,5-dihydrothiazolyl, pyridinyl, oxazolyl, isoxazolyl, imidazolyl, pyrimidyl, tetrazolyl, thienyl, benzothiazolyl and phenyl, possibly substituted by one or more radicals chosen from the group constituted by a) the halogens, b) the alkyl radicals containing 1–4 carbon atoms unsubstituted or substituted by an amino, alkylamino or dialkylamino radical, of which the alkyl radicals contain 1–3 carbon atoms, c) the phenyl radical, d) the alkoxy radicals containing 1–4 carbon atoms, e) the hydroxy radical, f) the trifluoromethyl, radical, and g) the nitro radical, or $R_1'$ and $R_2'$ together form any one of the saturated or unsaturated rings mentioned above for $R_2'$, the said ring then being linked to the nitrogen atom by a double bond, $R_3$ represents a hydrogen atom, a halogen atom, an alkyl radical containing 1–4 carbon atoms, $R_4$ represents a hydrogen atom or a halogen atom, $R_5$ represents a halogen atom, providing that $R_3$, $R_4$ and $R_5$ do not each represent, at the same time, a fluorine atom, and $R_6$ represents a hydrogen atom, an alkyl radical containing 1–8 carbon atoms or an acyl radical containing 2–8 carbon atoms, as well as their salts of addition with acids and bases, characterized in that

– either a compound with the formula (II):

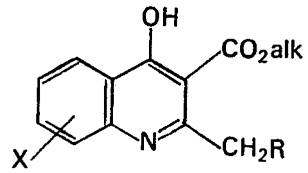

(II)

in which X retains the same significance as previously, alk represents an alkyl radical containing 1–8 carbon atoms and R represents a hydrogen atom or an alkyl radical containing 1–4 carbon atoms, is submitted to the action of a halogenation agent, then if applicable, to the action of a second halogenation agent, different from the first, so as to obtain the compound with the formula ($III_A$):

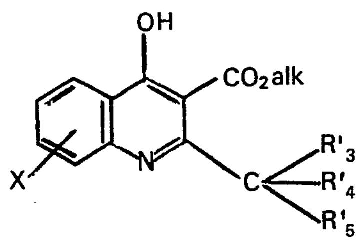

($III_A$)

in which $R_3'$, $R_4'$ and $R_5'$ retain the same significance given for $R_3$, $R_4$, and $R_5$ previously, which is submitted, if desired, to the action of a compound with the formula $Hal_1M$, in which $Hal_1$ represents a halogen atom and M an alkali metal atom, so as to obtain the compound with the formula ($III_B$):

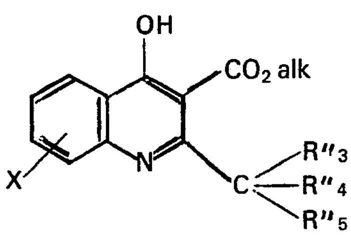

($III_B$)

in which $R_3''$ represents a hydrogen atom, a $Hal_1$ halogen atom, an alkyl radical containing 1–4 carbon atoms, $R_4''$ represents a hydrogen atom or a $Hal_1$ halogen atom and $R_5''$ represents a $Hal_1$ halogen atom, then the compound with the formula ($III_A$) or the compound with the formula ($III_B$) is submitted to the action of a saponification agent, so as to obtain a corresponding acid with the formula (IV):

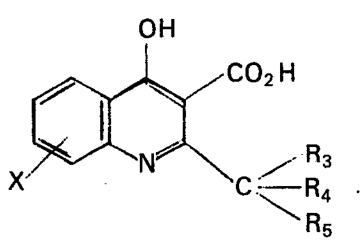

(IV)

in which $R_3$, $R_4$ and $R_5$ retain their preceding significance, which is transformed, if desired, into a functional derivative of the acid, then the acid with the formula (IV) or a functional derivative of this acid, is submitted to the action of a compound with the formula (V):

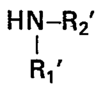

(V)

in which $R_1'$ and $R_2'$ retain their preceding significance, so as to obtain the corresponding compound with the formula ($I_A'$):

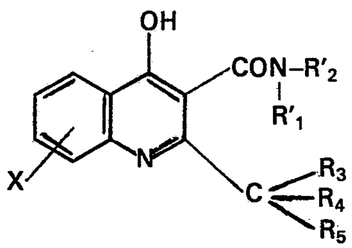

($I'_A$)

in which X, $R_1'$, $R_2'$, $R_3$, $R_4$ and $R_5$ are defined as previously, corresponding to a product with the formula (I') in which $R_6$ represents a hydrogen atom, which product with the formula ($I_A'$) is submitted, if desired, to the action of an etherification agent or an esterification agent, so as to obtain a product with the formula ($I_B'$):

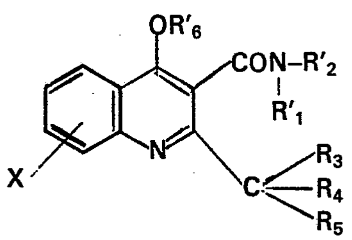

($I'_B$)

in which X, $R_1'$, $R_2'$, $R_3$, $R_4$ and $R_5$ are defined as previously, and $R_6'$ has the previously indicated values for $R_6$, with the exception of hydrogen, which product with the formula ($I_A'$) or ($I_B'$) is submitted, if desired, to the action of an acid or a base so as to form its salt, or a compound with the formula (VI):

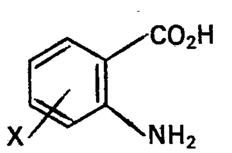

(VI)

in which X retains its significance given in Claim 1, is submitted to the action of an acid with the formula (VII):

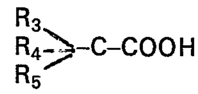

(VII)

in which $R_3$, $R_4$, $R_5$ retain their significances given in Claim 1 or a functional derivative of the acid with the formula (VII), so as to obtain a compound with the formula (VIII):

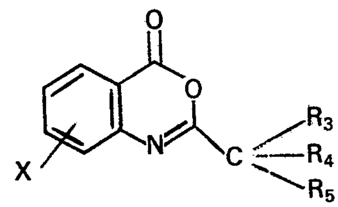

(VIII)

in which X retains its preceding significance, which is submitted to the action of a compound with the formula (IX):

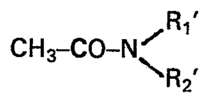

(IX)

in which $R_1'$ and $R_2'$ retain the significances given in Claim 1, so as to obtain the compound with the formula (X):

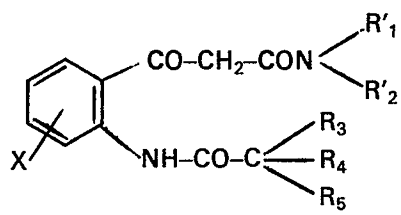

(X)

in which $R_1'$, $R_2'$, $R_3$, $R_4$ and $R_5$ retain their preceding significances, which is cyclized in the presence of an alkaline agent, so as to obtain a compound with the formula ($I_A'$), as previously defined, which compound with the formula ($I_A'$) is submitted, if desired, to the action of an etherification agent or an esterification agent, so as to obtain a compound with the formula ($I_B'$), as previously defined, which compound with the formula ($I_A'$) or ($I_B'$), if desired, is submitted to the action of an acid or a base so as to form its salt.

2. Process according to Claim 1, for preparing the compounds with the formula (I') as defined in Claim 1, characterized in that a compound with the formula (II):

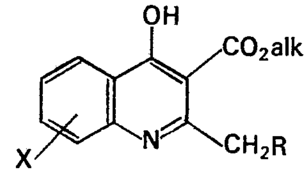

(II)

in which X retains the same significance as previously, alk represents an alkyl radical, containing 1–8 carbon atoms and R represents a hydrogen atom or an alkyl radical containing 1–4 carbon atoms, is submitted to the action of a halogenation agent, then if applicable to the action of a second halogenation agent different from the first, so as to obtain the compound with the formula ($III_A$):

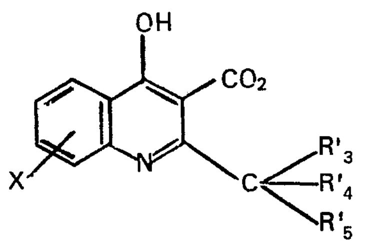

($III_A$)

in which $R_3'$, $R_4'$ and $R_5'$ retain the significance given for $R_3$, $R_4$ and $R_5$ previously, which is submitted, if desired, to the action of a compound with the formula $Hal_1M$, in which $Hal_1$ represents a halogen atom and M an alkali metal atom, so as to obtain the compound with the formula ($III_B$):

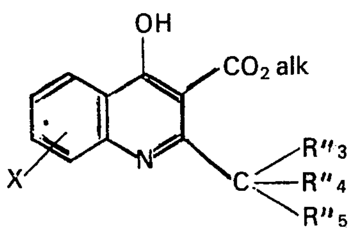

($III_B$)

in which $R_3''$ represents a hydrogen atom, a $Hal_1$ halogen atom, an alkyl radical containing 1–4 carbon atoms, $R_4''$ represents a hydrogen atom or a $Hal_1$ halogen atom and $R_5''$ represents a $Hal_1$ halogen atom, then the compound with the formula ($III_A$) or the compound with the formula ($III_B$) is submitted to the action of a saponification agent, so as to obtain a corresponding acid with the formula (IV):

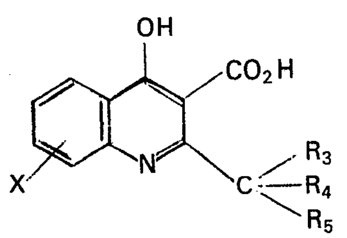

(IV)

in which $R_3$, $R_4$ and $R_5$ retain the preceding significance, which, if desired, is transformed into a functional derivative of the acid, then the acid with the formula (IV) or a functional derivative of this acid is submitted to the action of a compound with the formula (V):

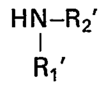

(V)

in which $R_1'$ and $R_2'$ retain their preceding significance so as to obtain the compound with the formula ($I_A'$):

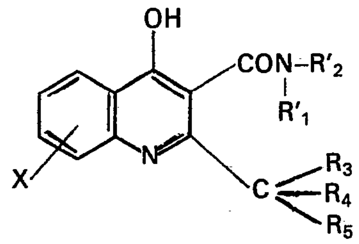

($I'_A$)

in which X, $R_1'$, $R_2'$, $R_3$, $R_4$ and $R_5$ are defined as previously, corresponding to a product with the formula (I') in which $R_6$ represents a hydrogen atom, which product with the formula ($I_A'$) is submitted, if desired, to the action of an etherification or an esterification agent, so as to obtain a product with the formula ($I_B'$):

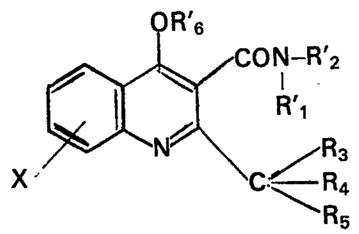

($I'_B$)

in which X, $R_1'$, $R_2'$, $R_3$, $R_4$ and $R_5$ are defined as previously and $R_6'$ has the previously indicated values for $R_6$, with the exception of hydrogen, which product with the formula ($I_A'$) or ($I_B'$), if desired, is submitted to the action of an acid or a base so as to form its salt.

3. Process according to Claim 1, for preparing the compounds with the formula (I'), as defined in Claim 1, characterized in that a compound with the formula (VI):

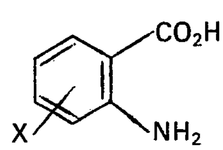

(VI)

in which X retains its previous significance, is submitted to the action of an acid with the formula (VII):

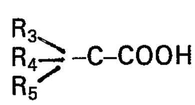

(VII)

in which $R_3$, $R_4$, $R_5$ retain their significances given previously or a functional derivative of the acid with the formula (VII), so as to obtain a compound with the formula (VIII):

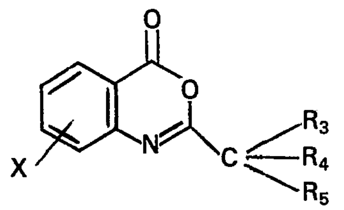

in which X retains its preceding significance, which is submitted to the action of a compound with the formula (IX):

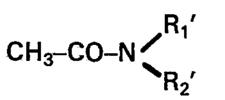

(IX)

in which $R_1'$ and $R_2'$ retain their significances given previously, so as to obtain the compound with the formula (X):

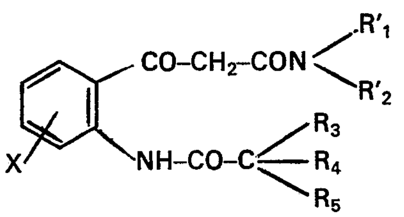

(X)

in which $R_1'$, $R_2'$, $R_3$, $R_4$ and $R_5$ retain their preceding significances, which is cyclized in the presence of an alkaline agent, so as to obtain a compound with the formula ($I_A'$), as defined previously, which compound with the formula ($I_A'$) is submitted, if desired, to the action of an etherification or esterification agent, so as to obtain a compound with the formula ($I_A'$) or ($I_B'$) which, if desired, is submitted to the action of an acid or a base so as to form its salt.

4. Process according to Claim 3, characterized in that the functional derivative of an acid with the formula (VII) is a halide or an anhydride, the reaction between the compound with the formula (VIII) and the compound with the formula (IX) takes place in the presence of an organo-lithium compound or a lithium amide.

5. Process according to Claim 2, for preparing the compounds with the formula (I):

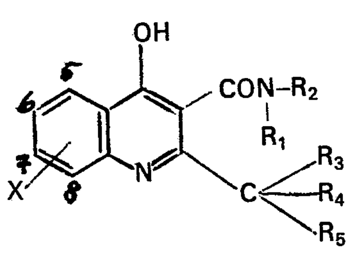

(I)

in which X is defined as in Claim 1, $R_1$ represents a hydrogen atom or an alkyl radical containing 1–4 carbon atoms, $R_2$ represents a radical chosen from the following radicals: thiazolyl, 4,5- dihydrothiazolyl, pyridinyl, oxazolyl, isoxazolyl, imidazolyl, pyrimidyl or tetrazolyl, possibly substituted by an alkyl radical containing 1–4 carbon atoms, and phenyl, possibly substituted by at least one radical chosen from the group formed by the hydroxy radicals, the alkyl radicals containing 1–4 carbon atoms, the alkoxy radicals containing 1–4 carbon atoms, the trifluoromethyl radical, the nitro radical and the halogen atoms and $R_3$, $R_4$ and $R_5$ are defined as in Claim 1, as well as their salts of addition with acids and bases, characterized in that the compound with the formula ($III_A$) or the compound with the formula ($III_B$) is submitted to the action of a saponification agent, so as to obtain a corresponding acid with the formula (IV):

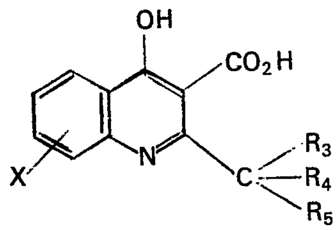

(IV)

in which $R_3$, $R_4$ and $R_5$ retain their preceding significance, which, if desired, is transformed into a functional derivative of the acid, then the acid with the formula (IV) or a functional derivative of this acid is submitted to the action of a compound with the formula (V'):

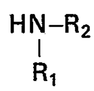

(V')

in which $R_1$ and $R_2$ are defined as previously, so as to obtain the compound with the formula (I), which, if desired, is submitted to the action of an acid or a base so as to form its salt.

6. Process according to Claim 2, characterized in that a compound with the formula ($II_a$):

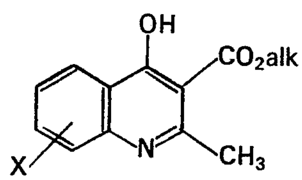

($II_a$)

in which X and alk retain their significance indicated in Claim 2, is submitted to the action of a halogenation agent, so as to obtain the compound with the formula ($III_a$):

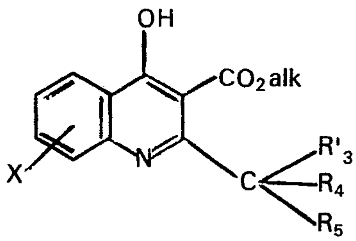

($III_a$)

in which $R_3'$ and $R_4$ either represent a hydrogen atom, or the same halogen atom as $R_5$, which product ($III_a$) is submitted to the action of a saponification agent, so as to obtain a compound with the formula ($IV_a$):

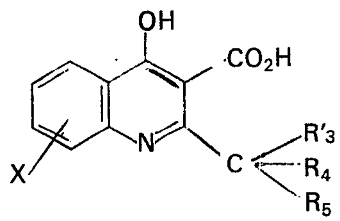

($IV_a$)

which is transformed, if desired, into a functional derivative of the acid, then either the acid with the formula ($IV_a$), or a functional derivative of this acid is submitted to the action of a compound with the formula (V):

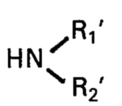

(V)

in which $R_1'$ and $R_2'$ retain their preceding significance, so as to obtain a compound with the formula ($I_C'$):

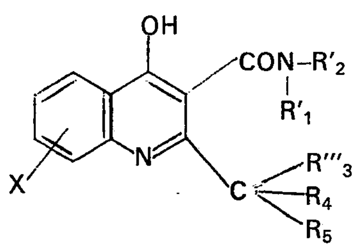

($I'_c$)

in which X, $R_1'$, $R_2'$, $R_3'''$, $R_4$ and $R_5$ are defined as previously, corresponding to a product with the formula (I') in which $R_6$ represents a hydrogen atom and $R_3$ has the values of $R_3'''$, which product with the formula ($I_C'$) is submitted, if desired, to the action of an etherification or esterification agent, so as to obtain a product with the formula ($I_D'$):

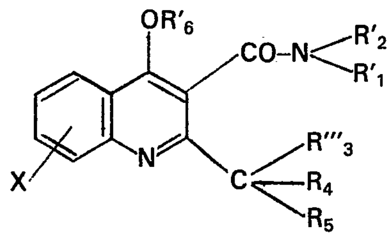

($I'_D$)

in which X, $R_1'$, $R_2'$, $R_3'''$, $R_4$ and $R_5$ are defined as previously and $R_6'$ has the previously indicated values for $R_6$, with the exception of hydrogen, which product with the formula ($I_C'$) or ($I_D'$) is submitted, if desired, to the action of an acid or a base, so as to form its salt.

7. Process according to Claim 6, for preparing the compounds corresponding to the formula (I), as defined in Claim 4, as well as their salts, characterized in that a compound with the formula ($II_a$):

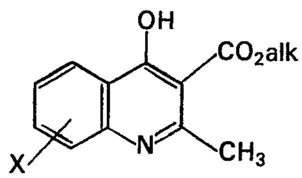

($II_a$)

in which X and alk retain their significance indicated in Claim 1, is submitted to the action of a halogenation agent, so as to obtain the compound with the formula ($III_a$):

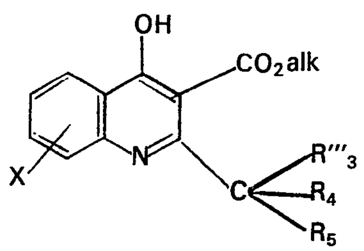

($III_a$)

in which $R_3'''$ and $R_4$ each represent either a hydrogen atom or the same halogen atom as $R_5$, which product with the formula ($III_a$) is submitted to the action of a saponification agent so as to obtain a compound with the formula ($IV_a$):

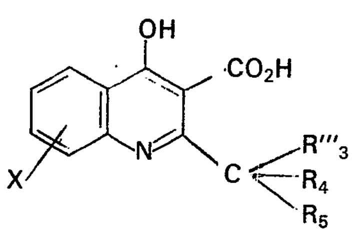

($IV_a$)

which, if desired, is transformed into a functional derivative of the acid, then either the acid with the formula ($IV_a$), or a functional derivative of this acid, is submitted to the action of a compound

with the formula (V') in which $R_1$ and $R_2$ are defined as previously, so as to obtain the compound with the formula (I), which, if desired, is submitted to the action of an acid or a base so as to form its salt.

8. Process according to any one of Claims 2, 5, 6 or 7, characterized in that the halogenation agent utilized is N-chloro-or N-bromosuccinimide.

9. Process according to Claim 2 or 6, characterized in that the compound with the formula ($III_a$), ($III_B$) or ($III_a$) ist submitted to the action of a compound with the formula (V):

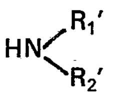

(V)

in which $R_1'$ and $R_2'$ are defined as in Claim 2, in the presence of a trialkylaluminium, so as to obtain a compound with the formula ($I_A'$) or ($I_C'$), as defined in Claims 2 or 6, then, if desired the synthesis is continued as described in Claim 2 or 6.

10. Process according to any one of Claim 1–9, characterized in that a compound with the formula (II), ($II_a$) or (VI) in which X represents a trifluoromethyl radical is utilized at the start.

11. Process according to any one of Claims 1–9, characterized in that a compound with the formula (V), (V') or (IX) in which $R_1'$ or $R_1$ represents a hydrogen atom is utilized at the start.

12. Process according to any one of Claims 1–9, characterized, in that a compound with the formula (V), (V') or (IX) in which $R_2'$ or $R_2$ represents a thiazolyl radical is used at the start.